# EUROPEAN PATENT APPLICATION

(11) **EP 4 556 909 A1**
(43) Date of publication of application: **21.05.2025**
(21) Application number: 23383183.3
(22) Date of filing: 20.11.2023
(51) Int. Cl.: G01N 33/574

(54) **AN IN VITRO METHOD FOR DETECTING CANCER**

(71) Applicant: Inmunotek, S.L., 28805 Alcalá de Henares (ES); Fundación Investigación del Hospital Clínico San Carlos Madrid, 28040 Madrid (ES)
(72) Inventor: Subiza Garrido-Lestache, José Luis, Madrid (ES); Tudela Garcia, José Ignacio, Madrid (ES); Moltó Delgado, Luis Manuel, Madrid (ES); Fernández Arquero, Miguel, Madrid (ES); Viñuela Martín, Marcos, Madrid (ES); Cañada Vicinay, Francisco Javier, Madrid (ES); Kalograiaki, Ioanna, Tesalónica (GR); Palomares Gracia, Oscar, Madrid (ES); Martín de la Cruz, Leticia, Madrid (ES); Angelina Querencias, Alba, Madrid (ES)
(74) Representative: Pons

(57) **Abstract**

The present invention relates to an *in vitro* method for detecting cancer in a subject, for predicting the clinical outcome of a subject suffering from cancer, or for monitoring the treatment of a subject suffering from cancer, based on the tumoral carbohydrate Ca10 (Ca10) levels in a sample isolated from the subject.

## Description

The invention refers to an *in vitro* method for cancer detection based on the tumor-associated carbohydrate Ca10 (Ca10) levels in a biological sample. Thus, the present invention relates to the technical field of cancer, specifically, to tumor markers thereof.

### BACKGROUND ART

Compelling experimental evidence demonstrated the involvement of the immune system in both tumor rejection and tumor progression. Effective host antitumor responses are challenged by tumor escape mechanisms based on suppression of effector cells. Regulatory T cells (Tregs) are a heterogeneous group of T cells with potent suppressive capacity that are classified as: thymus-derived CD25+FOXP3+ Tregs and peripherally-induced Tregs generated outside the thymus upon antigenic stimulation. Tregs are indispensable to keep tissue homeostasis in many biological contexts such as autoimmunity, allergy, metabolic syndrome, transplantation, infection, or cancer. Functional Tregs are abnormally expanded in cancer and greatly increased within the tumor tissues, playing a key role in tumor development and progression and posing a major barrier to anti-cancer immunity. In tumor tissues, Tregs can be generated from local FOXP3⁻ conventional T cells and/or recruited to the tumor site upon previous expansion in lymphoid organs. The mechanisms by which Tregs are fueled in hosts bearing malignant tumors and the contribution of the tumor microenvironment (TME) to these processes are not yet fully understood. In this regard, dendritic cells (DCs) are professional antigen-presenting cells crucial in the orchestration of proper immune responses by linking innate and adaptive immunity. DCs recognize all surrounding antigens within the tissues, which are then processed and presented to T cells to initiate proper adaptive immune responses depending on the inflammatory context and surrounding environmental cues. Under homeostatic non-inflammatory conditions, immature DCs remain tolerogenic and generate functional Tregs. The ability of mature DCs infiltrating tumors to promote highly suppressive Tregs is strongly influenced by exogenous signals encountered in the TME. However, how DCs integrate these signals and the functional resulting outcomes remains elusive.

Malignant transformation is accompanied by changes in tumor cell surface glycoconjugates, including aberrant or incomplete glycosylation of heavily glycosylated structures such as mucins and proteoglycans. These structures are often overexpressed and shed from tumor cells, having a strong influence on tumor progression by modulating cell adhesion, neo-angiogenesis and metastases. In addition, tumor-associated carbohydrates may inhibit anti-cancer immunity through different mechanisms such as the expansion of myeloid-derived suppressor cells, or the activation of Tregs.

Murine Ehrlich's tumor (ET) cells, originally derived from a spontaneous mammary carcinoma, lack of H-2 antigens and can grow either in ascitic or solid form in almost any mouse strain across different antigenic backgrounds, representing a suitable model to uncover potential novel tumor escape mechanisms. The carbohydrate Ca10 is a highly glycosylated ET cell surface macromolecule initially defined by the binding of monoclonal antibody A10. Ca10 is spontaneously released from ET cells and can be detected in the serum of solid ET-bearing mice. A10 also reacts with glycan moieties located in some human cancer mucins, but the structural features of the carbohydrate moiety contained in Ca10 and its potential functional implications in cancer remains largely unknown.

Therefore, there is the necessity in the state to the art to increase the insights on tumor escape mechanisms in order to develop new tools in the detection and treatment of cancer.

### DESCRIPTION OF THE INVENTION

By studying the presence of regulatory T cells (Tregs) in Murine Ehrlich carcinoma (mammary tumor of spontaneous origin that grows in immunocompetent mice), it has been possible to establish the relationship between the increase of said Tregs in the spleen and the size of the tumor, and importantly, also with the serum levels of murine Ca10 (mCa10), a circulating carbohydrate released by Ehrlich cells. mCa10 is defined by the monoclonal antibody A10, generated against carbohydrates on the cell surface of Ehrlich tumor cells. Surprisingly, exogenous administration of purified mCa10 induces the generation of Tregs in the spleen of tumor-free mice in a dose-dependent manner. The mechanism of induction of Tregs by mCa10 has been studied *in vitro.* Its interaction with DCs promotes functional changes in these cells towards a tolerogenic phenotype that induces the differentiation of naive T cells into functional Tregs with a strong suppressor capacity.

mCa10 is a proteoglycan with a heparan sulfate glycosaminoglycan structure, sensitive to certain heparinases (heparinase 3). The heparan sulfate structure found in mCa10 is peculiar for its extremely low sulfatation degree. It would be related to the non-sulfated precursor of this glycosaminoglycan, heparosan, normally absent on the cell surface of mammalian cells. The analysis of the main carbohydrate component in mCa10, either by nuclear magnetic resonance (NMR) or by chromatography of the released fragments after complete hydrolysis with heparinases only shows a significant proportion (20-40%) of non-acetylated glucosamine residues. It does not show sulfation of either glucosamine or glucuronic acid residues, contrary what could be expected for normally processed heparan sulfate glycosaminoglycan present in healthy tissues. This discrepancy suggests an atypical processing or modification in the mCa10 heparan sulfate glycosaminoglycan.

Surprisingly, circulating levels of a human homologue of the murine tumor carbohydrate Ca10 (Ca10H) are detected in the serum of cancer patients with tumors originating from different tissues. The structural relationship between the two murine and human carbohydrates is based on the fact that both are recognized by the same monoclonal antibody A10, but also on their sensitivity to heparinase 3. Ca10H levels are found to be elevated in cancer patients, on a cut-off established with sera from control subjects. Of note, in patients with prostate cancer, it has been possible to establish a correlation between serum Ca10H levels and bone metastases.

Overall, it follows that induction of Tregs by Ca10 may be an important tumor escape mechanism in Ehrlich tumor and possibly also in human cancer. Since Ca10H levels are variable in different patients with the same histological type, Ca10H could represent a new "tumor marker" with clinical usefulness as a prognostic factor. On the other hand, Ca10H could itself be a therapeutic target in order to neutralize tumor-induced generation and expansion of Tregs. As the occurrence of Ca10H is not restricted to a single histological type of tumor, the potential benefit of a therapy aimed at such neutralization could encompass a wide number of tumors. Moreover, the fact that Ca10H can be detected in circulation would facilitate the selection of those tumors amenable to treatment as a companion diagnostic tool.

### Uses of Ca10 as a tumor marker.

In view of the foregoing, the present invention discloses Ca10H as a "tumor marker", this is, a substance that is found in high levels in biological sample of some people with cancer in comparison with non-suffering cancer people. Tumor markers are produced either by the cancer cells themselves or by the body in response to the presence of cancer or certain benign (noncancerous) conditions.

Based on the above, a first aspect the present invention relates to an *in vitro* method for detecting cancer in a subject, hereinafter "detection method of the invention", comprising:
(a) quantifying the tumoral carbohydrate Ca10 levels in a biological sample isolated from the subject; and
(b) comparing the Ca10 levels obtained in step (a) with a control level,

Wherein if the Ca10 levels in the subject are increased with respect to the control levels, then the subject suffers from cancer.

As used herein, the term "cancer detection" refers to the identification of a tumoral tissue by examination or parameters or biomarkers in a subject. In the context of the present invention, the parameter required for detecting cancer is the Ca10H levels in a sample of the subject.

As use herein, the term "cancer" refers to the disease resulting from the uncontrolled proliferation of cells giving rise to a malignant tumour or to a malignant tumoral tissue. A tumour is considered malignant when the tumour cells can grow rapidly, show anaplasia and/or are able to infiltrate the tissue, invade adjacent tissues or even spread to other parts of the body, a process known as metastasis. Thus, in one particular embodiment, the cancer is a metastatic cancer.

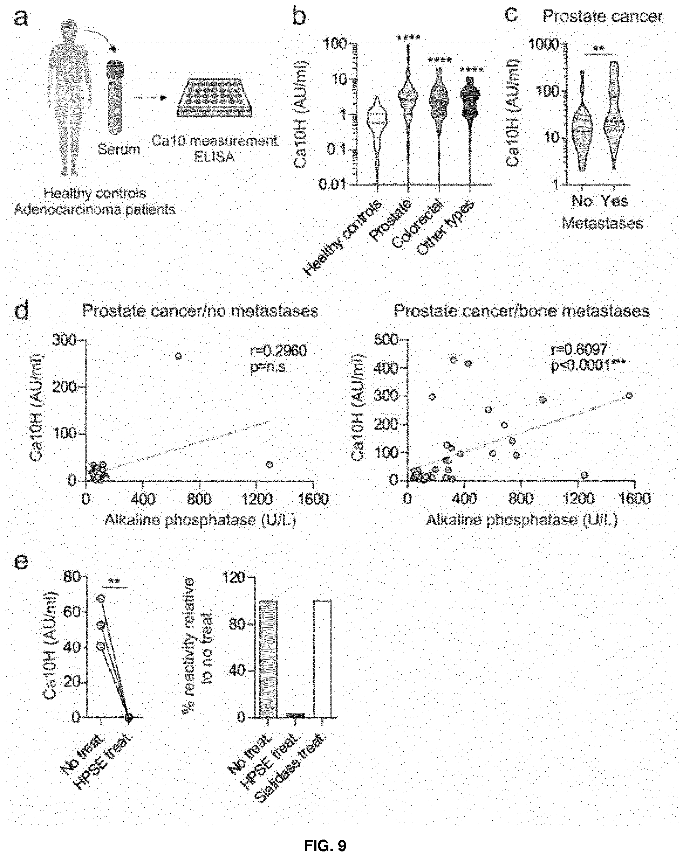

On the other hand, the tumour (or cancer) can be located or originate in any tissue or organ of the body. Thus, any cancer is susceptible to be identified by the detected method of the invention, regardless of its stage of development, its origin or its location. There are several main types of cancer. Carcinoma is a cancer that begins in the skin or in tissues that line or cover internal organs. Sarcoma is a cancer that begins in bone, cartilage, fat, muscle, blood vessels, or other connective or supportive tissue. Leukemia is a cancer that starts in blood-forming tissue, such as the bone marrow, and causes large numbers of abnormal blood cells to be produced and enter the blood. Lymphoma and multiple myeloma are cancers that begin in the cells of the immune system. Central nervous system cancers are cancers that begin in the tissues of the brain and spinal cord. The cancer may be a solid tumor or a non-solid tumor, including metastasis. In a preferred embodiment, patients diagnosed with selected cancers, such as carcinomas and adenocarcinomas, had on average above five times higher levels of Ca10H than healthy controls. Specifically, the mean levels of Ca10H in these patients was 4.0 Arbitrary Units (AU)/mL with a standard error of the mean (SEM) of 0.6, compared to a mean of 0.7 AU/mL with a SEM of 0.0 in the control group (see Figure 9b). Thus, in a particular embodiment, the cancer is a carcinoma or an adenocarcinoma.

Examples of cancer include, but are not limited to, a lung tumor or cancer, such as a small cell or non-small cell lung cancer, or an adenocarcinoma, squamous cell carcinoma or a large cell carcinoma. Further particular non-limiting examples of a tumor or cancer include a carcinoma, sarcoma, lymphoma, leukemia, adenoma, adenocarcinoma, melanoma, glioma, glioblastoma, meningioma, neuroblastoma, retinoblastoma, astrocytoma, oligodendrocytoma, mesothelioma, reticuloendothelial, lymphatic or haematopoietic neoplasia, tumor, cancer or malignancy. Additional particular non-limiting examples of tumor or cancer is a lung, thyroid, head or neck, nasopharynx, throat, nose or sinuses, brain, spine, breast, adrenal gland, pituitary gland, thyroid, lymph, gastrointestinal (mouth, esophagus, stomach, duodenum, ileum, jejunum (small intestine), colon, rectum), genito-urinary tract (uterus, ovary, cervix, endometrial, bladder, testicle, penis, prostate), kidney, pancreas, liver, bone, bone marrow, lymph, blood, muscle, or skin neoplasia, tumor, or cancer. Still further particular non-limiting examples of a tumor or cancer include a breast cancer, prostate cancer, pancreas cancer, gastric cancer, pleural mesothelioma, colon cancer, rectal cancer, large bowel cancer, small intestinal cancer, esophageal cancer, duodenal cancer, lingual cancer, pharyngeal cancer, salivary gland cancer, cerebral tumor, schwanoma, liver cancer, kidney cancer, bile duct cancer, endometrial cancer, cervical cancer, uterine body cancer, ovarian cancer, bladder cancer, urethral cancer, skin cancer, angioma, malignant lymphoma, malignant melanoma, thyroid cancer, parathyroid cancer, nasal cancer, paranasal cancer, auditory organ cancer, carcinoma of oral floor, laryngeal cancer, parotid cancer, submandibular cancer, bone tumor, angiofibroma, retinal sarcoma, penile cancer, testicular tumor, pediatric solid cancer, Kaposi's sarcoma, tumor of maxillary sinus, fibrous histiocytoma, leiomyosarcoma, rhabdomyosarcoma, lymphoma, multiple myeloma or leukemia.

Nevertheless, in a particular embodiment of the detection method of the invention, alone or in combination with all or each one of the previous particular embodiments, the cancer is prostate cancer, cervix cancer, breast cancer, pancreatic cancer, lung cancer, gastric cancer, endometrial cancer, skin cancer, thyroid cancer, bladder cancer, ovarian cancer, liver cancer, esophageal cancer, or colorectal cancer.

The first step of the detection method of the invention comprises quantifying the Ca10 levels in a biological sample isolated from the subject.

The term "tumoral carbohydrate Ca10" or "Ca10" encompasses both the murine tumoral carbohydrate derived from Ehrlich's tumor cells (mCa10), as well as any other homologue tumoral carbohydrate equivalent to mCa10 in other mammal, such as its human counterpart, i.e. the human tumoral carbohydrate Ca10 or Ca10H.

The "tumoral carbohydrate Ca10" or "Ca10" refers to a proteoglycan with a heparan sulfate glycosaminoglycan structure, sensitive to certain heparinases (heparinase 3), that reacts with the monoclonal antibody A10 (Gil, J. et al. 1990. Cancer Res 50, 7301-7306). In particular, the mCa10 shows the following features:
The principal polysaccharide component of mCa10 is an incompletely processed heparosan, precursor of heparan sulfate, and hydrolysable with heparinases, with an average molecular size over 100 kDa, as measured by NMR diffusion ordered spectroscopy (DOSY) where N-acetylglucosamine residues are deacetylated between 20 to 40% and the total sulfation observed below 0,2% as measured by NMR and chromatographic methods coupled to mass spectrometry. The generic polymeric structure of mCa10 can be described as a polysaccharide comprising a combination of tetramers of formula: 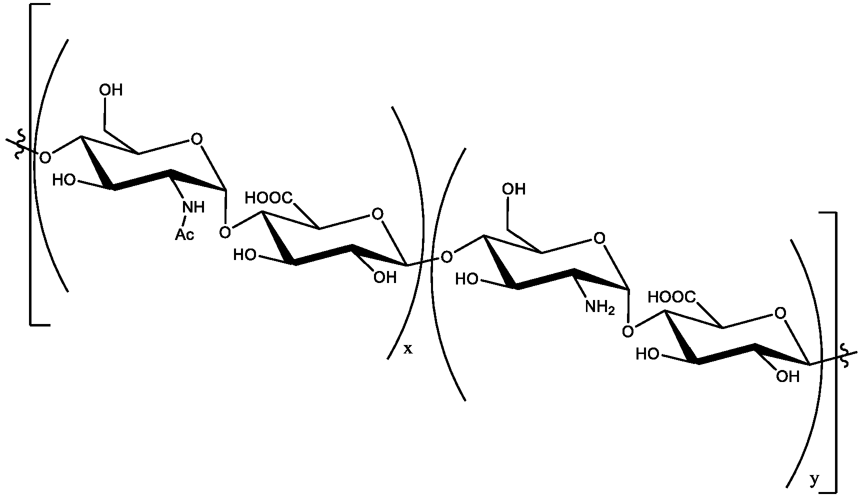 wherein each tetramer is
(a) x= 2 and y= 0; or
(b) x=1 and y= 1; or
(c) x=0 and y=2; and
wherein 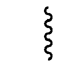 represents the point of attachment to another tetramer; and
wherein
The ratio Σx/Σy in the polysaccharide is from 4/1 to 3/2; and wherein
The total number of tetramers in the polysaccharide is from 50 to 150.

The polysaccharide may comprise attach to one of ends a group -OH, a residue beta-Glucuronyl or a glycosaminoglycan protein attachment sequence.

Thus, the main polysaccharide component of mCa10 is constituted by the disaccharide repeating units of heparosan, β-D-glucuronic acid (GlcA) and α-D-N-acetylglucosamine (GlcNAc), where a proportion between 20% to 40% of N-acetylglucosamine residues are deacetylated (GlcN), according to the simplified formula: [→4) β-D-GlcA (1 → 4)-α-D-GlcNAc/GlcN (1→] . The mCa10 comprising the polysaccharide of the above-mentioned formula is capable of inducing the production of Tregs. Without wishing to be bound by any theory, and based on the results shown in the present invention, the inventors consider that the human counterpart of mCa10, i.e. Ca10H, shows a similar structure to one of mCa10.

Methods for quantifying the levels or amount of carbohydrates in a sample are widely known in the state of the art, and any of them can be used to carry out step a) of the cancer detection method of the invention. Non-limiting examples of these methods include: Lectin based methods, NMR, chromatography, mass spectrometry and colorimetric methods depending of the nature of the carbohydrate components. For example, heparan-sulphate quantification and analysis in plasma samples can be performed by liquid chromatography coupled to mass spectrometry. Nevertheless, in a particular embodiment of the cancer detection method of the invention, alone or in combination with all or each one of the previous particular embodiments, the method used for quantifying the level of Ca10H is by ELISA (Enzyme-Linked ImmunoSorbent Assay), more particularly, by ELISA using the monoclonal antibody A10 (Gil, J. *et al.* 1990. Cited *ad supra*).

The cancer detection method of the present invention can be performed on any suitable biological sample. As used herein, a "biological sample" refers to any sample of biological material derived from an animal such as, but not limited to, cellular material, biofluids (eg. Blood, serum, plasma, pleural, bronchioalveolar and peritoneal liquids, etc.), faeces, urine, sputum and tissue biopsy. The biological sample may be tested directly or may require some form of treatment prior to testing or may be partially purified or otherwise enriched prior to analysis. Thus, in a particular embodiment, alone or in combination with all or each one of the previous particular embodiments, the biological sample is blood, serum, plasma, pleural liquid, bronchioalveolar liquid, peritoneal liquid, faeces, urine, sputum or tissue biopsy specimens.

Likewise, the cancer detection method of the present invention can be carried out on any subject. As used herein, the term "subject" refers to all animals classified as mammals and includes but is not limited to domestic and farm animals, primates and humans, for example, human beings, non-human primates, cows, horses, pigs, sheep, goats, dogs, cats, or rodents. In a particular embodiment of the cancer detection method of the invention, alone or in combination with all or each one of the previous particular embodiments, the subject is a mammal, more preferably, a human.

Next, in step b) of the detection method of the invention, the levels of Ca10 quantified in step a) are compared with the ones in a control level. In the context of the present invention, the "control level" refers to the levels of Ca10 in sample isolated from a subject not suffering from cancer or not comprising tumoral tissue. As the skilled person in the art understands, the control level may also refer to the median value of the Ca10 levels in a collection of biological samples from subjects not suffering from cancer or not comprising tumoral tissue. In this case, said control level is typically obtained by combining equal amounts of samples from a population of subjects who are clinically well documented. In the context of the present invention, the terms "control level" and "reference value" are equivalents and refer to the same concept.

In the context of the present invention, it is understood that the Ca10 levels are increased with respect to the control level when a value is greater or higher than the other. In particular, it is understood that the Ca10 levels are "increased" when the levels of the Ca10 in the subject sample are, at least, 1.1-fold, 1.5-fold, 2-fold, 5-fold, 10-fold, 20-fold, 30-fold, 40-fold, 50-fold, 60-fold, 70-fold, 80-fold, 90-fold, 100-fold higher, or even more, than the Ca10 levels in the control sample. Finally, depending on the result of said comparison, it can be concluded whether the subject suffers or not from cancer. In this way, if the Ca10 levels in the subject are increased with respect to the control level, then the subject suffers from cancer.

In another aspect, the present invention relates to an *in vitro* method for predicting the clinical outcome of a subject suffering from cancer, or for monitoring the treatment of a subject suffering from cancer, hereinafter "second method of the invention", comprising
(a) quantifying the Ca10 levels in a biological sample isolated from the subject; and
(b) comparing the Ca10 levels obtained in step (a) with a control level,
wherein if the Ca10 levels in the subject are increased with respect to the control level, then
- the clinical outcome of the subject is negative and either the subject is going to suffer from metastasis or the tumoral mass is going to increase; or
- the treatment is not being effective.

The terms "cancer" and "subject" have been defined un previous paragraphs, and said definitions together with their particular embodiments are applicable to the second method of the invention.

The term "prediction" is used herein to refer to the likelihood that a patient will have a particular clinical outcome. As will be explained later, the clinical outcome may be positive or negative. The predictive method of the present invention can be used clinically to make treatment decisions by choosing the most appropriate treatment modalities for any particular patient. The predictive method of the present invention is a valuable tool in predicting if a patient is likely to respond favorably to a treatment regimen. As used herein, the expression "clinical outcome" is understood as the expected course of a disease. It denotes the doctor's prediction of how a subject's disease will progress, and whether there is chance of recovery o recurrence.

As used herein, the expression "monitoring the treatment" refers to determine the response of a subject to a therapy, i.e. it refers to the assessment of the results of a therapy in a patient with cancer in response to a therapy. The response of the subject to the therapy may be negative (the subject does not respond to the therapy) or positive (the subject responds to the therapy).

A first step of the second method of the invention comprises quantifying the Ca10 levels in a biological sample isolated from the subject. The terms "Ca10", "biological sample", "subject" has been defined previously in the present description together with their particular embodiments. Likewise, methods for quantifying the level of Ca10 have been also disclosed in previous paragraphs.

Next, the second method of the invention comprises comparing the Ca10 levels obtained in the first step with a control level. When the second method of the invention refers to predict the clinical outcome of the subject, the term "control level" refers to the amount of Ca10 in a subject suffering from cancer but no suffering from metastasis. As the skilled person understands, the control level may also refer to the median value of the Ca10 levels in a collection of biological samples from subjects suffering from cancer (or comprising tumoral tissue) but no suffering from metastasis. In this case, said control level is typically obtained by combining equal amounts of samples from a population of subjects who are clinically well documented.

Alternatively, when the second method of the invention refers to monitor the treatment administered to a subject, the term "control level" refers to the Ca10 levels in the same subject previously to be treated with a therapy.

Once the Ca10 levels between the subject and the control level are compared, the skilled person in the art may conclude that if the Ca10 levels in the subject are increased with respect to the control level, then
- the clinical outcome of the subject is negative and either the subject is going to suffer from metastasis or the tumoral mass is going to increase; or
- the treatment is not effective.

Thus, in the context of the second method of the invention, the subject has "negative clinical outcome" (or "the clinical outcome is negative" or "has poor prognosis") when the subject is going to suffer from metastasis, i.e. the cancer is going to evolve to metastasis (the tumor of the subject is a malignant tumor which is going to undergo metastasis), or the tumoral mass in going to increase in size, i.e. the tumor keep growing unaffected by the therapy, likely undergoing metastasis and reaching other organs.

On the other hand, as used in the second method of the invention, the expression "the treatment is not effective" means that the subject is not responding to the therapy, i.e. despite of the treatment administered to the subject, the cancer has evolved to metastasis, or the tumoral mass has increased in size. In the context of the second method of the invention, the expression "the tumoral mass has increased in size" means that the tumoral mass after the therapy is stabilized or the tumour keep growing.

In another aspect, the present invention relates to the use of a kit for detecting cancer *in vitro,* or for predicting *in vitro* the clinical outcome of a subject suffering from cancer, or for monitoring *in vitro* the treatment of a subject suffering from cancer, wherein the kit comprises elements for quantifying the Ca10 levels in a biological sample of a subject.

The terms "cancer", "biological sample", "subject", "prediction", "clinical outcome", "monitoring", etc. have been defined previously, and both their definition as well as their particular embodiments are applicable to the use of a kit for detecting cancer *in vitro.*

As used in the present invention, the term "Kit" refers to a product comprising the different reagents or elements necessary to carry out the detection method of the invention, or to quantify the Ca10 levels in a biological sample of a subject which are packaged to enable them to be transported and stored.

Therefore, in another particular embodiment of the use of a kit for the *in vitro* detection of cancer, or for predicting *in vitro* the clinical outcome of a subject suffering from cancer, or for monitoring *in vitro* the treatment of a subject suffering from cancer, the cancer is a carcinoma or an adenocarcinoma.

In another particular embodiment of the use of a kit for the *in vitro* detection of cancer, or for predicting *in vitro* the clinical outcome of a subject suffering from cancer, or for monitoring *in vitro* the treatment of a subject suffering from cancer, the cancer is prostate cancer, cervix cancer, breast cancer, pancreatic cancer, lung cancer, gastric cancer, endometrial cancer, skin cancer, thyroid cancer, bladder cancer, ovarian cancer, lever cancer, esophageal cancer, or colorectal cancer.

In another particular embodiment of the use of a kit for the *in vitro* detection of cancer, or for predicting *in vitro* the clinical outcome of a subject suffering from cancer, or for monitoring *in vitro* the treatment of a subject suffering from cancer, the biological sample is blood, serum or plasma pleural liquid, bronchioalveolar liquid, peritoneal liquid, faeces, urine, sputum or tissue biopsy specimens

Examples of reagents or elements for detecting Ca10H or quantifying the Ca10H level in a biological sample of a subject include, without being limited to, an antibody, in particular, anti-Ca10H antibodies; Lectins or structures designed to bind to characteristic Ca10H sites ([→4) β-D-GlcA (1→4)-α-D-GlcNAc/GlcN (1→].), Fv fragments, nanobodies, peptides and aptamers, all of them with affinity for Ca10H.

In another particular embodiment of the use of a kit for the *in vitro* detection of cancer, or for predicting *in vitro* the clinical outcome of a subject suffering from cancer, or for monitoring *in vitro* the treatment of a subject suffering from cancer, the elements are antibodies, or a fragment thereof; peptides; or aptamers; all of them with affinity for Ca10H.

The kit may further include, without any limitation, buffers, agents to prevent contamination, inhibitors of protein degradation, etc. Suitable materials for packaging the components of the kit include glass, plastic (polyethylene, polypropylene, polycarbonate and the like), bottles, vials, paper, sachets and the like. Additionally, the kits of the invention may contain instructions for simultaneous, sequential or separate use of the different components in the kit. Said instructions may be in the form of printed material or in the form of an electronic medium capable of storing instructions so that they can be read by a subject, such as electronic storage media, optical media and the like.

### Method of treatment of the invention.

The inventors of the present invention have also observed that Ca10 could itself be a therapeutic target in order to neutralize tumor-induced generation and expansion of Tregs. As the occurrence of Ca10 is not restricted to a single histological type of tumor, the potential benefit of a therapy aimed at such neutralization could encompass a wide number of tumors.

Therefore, in another aspect, the present invention relates to a Ca10 inhibitor, or a composition or kit comprising it, for use in the treatment of cancer in a subject, wherein the Ca10 inhibitor is a molecule which (i) specifically binds Ca10 and (ii) is able to impair or to inhibit the generation of Tregs. Hereinafter "Ca10 inhibitor of the invention for treating cancer".

The terms "cancer", "Ca10" and "subject" have been defined above for previous inventive aspects, and they are applicable to the present inventive aspect along with their particular embodiments. The structure of mCa10 has also been disclosed above in the present description.

As used herein, the term "Ca10 inhibitor" refers to any molecule or compound capable of capable of completely or partially inhibiting the activity of Ca10, i.e.: the molecule or compound blocks or prevents the induction of Tregs (regulatory T cells) by Ca10. Therefore, a Ca10 inhibitor will be characterised by preventing or decreasing the tumor-induced immune suppression.

Ca10 inhibitors include, without being limited to, an antibody, in particular anti-Ca10H antibodies, Fv fragments, a nanobody, a peptide, an aptamer, lectins, or structures designed to bind to characteristic Ca10 sites ([→4) β-D-GlcA (1 → 4)-α-D-GIcNAc/GIcN (1→]). In addition to blockade by binding to Ca10, Ca10 inhibition can also be achieved by degradation of Ca10 with specific enzymes, such as heparinases, also named heparitinases, and heparanases (endoglucuronidases), active on heparan-sulfate related polysaccharides. Thus, in a particular embodiment of the Ca10 inhibitor of the invention for treating cancer, the Ca10 inhibitor is an antibody, a Fv fragments, a nanobody, a peptide, an aptamer or an enzyme. Assays to identify inhibitors of Ca10H acting by blockade or degradation include, but are not limited to: immunoprecipitation, radioimmunoassay, immunofluorescence assay, enzyme immunoassay, chemiluminescent assay, enzyme-linked immunosorbent assay (ELISA), or enzymatic degradation assays. Functional assays evaluating the effect of Ca10H blockade or degradation can be performed on *in vitro* cell cultures with dendritic or myeloid cells, as those disclosed in the examples of the present description.

In the context of the present invention, "antibody" is understood as any antibody capable of binding specifically to Ca10, and blocking or inhibiting one or more of the functions of Ca10, being one of said functions the induction of Tregs. The antibodies capable of binding to Ca10, specifically recognize the saccharide portion of the Ca10, i.e. glucosamine residues with non-acetylated free amino acid groups within Ca10, as shown in the above formula.

Antibodies can be prepared using any of the methods that are known to the person skilled in the art. Techniques for preparing and using various antibody-based constructs and fragments are well known in the state of the art. Means for preparing and characterising antibodies are also known in the state of the art. In the context of the present invention, suitable antibodies include intact antibodies comprising a variable antigen binding region and a constant region, "Fab", "F(ab')2" and "Fab"', Fv, scFv fragments, diabodies and bispecific antibodies, all of them capable of binding specifically to Ca10, and blocking or inhibiting one or more of the functions of Ca10. Once the antibodies with binding capacity to Ca10 are identified, those capable of inhibiting the activity of this molecule will be selected by using an assay for identifying inhibition assays, as the one disclosed in the example of the present description. Examples of antibodies include, without limiting to, monoclonal antibody A10 (Gil, J. et al.1990. Cancer Res 50, 7301-7306), and monoclonal antibodies that react with deacetylated glucosamine residues of heparosan, such as JM403 (commercialised by AMSBIO).

The Ca10 inhibitor for use as described herein may be comprised within a composition, particularly, a pharmaceutical composition. In another particular embodiment, the Ca10 inhibitor is in a therapeutically effective amount, and optionally said pharmaceutical composition further comprises a pharmaceutically acceptable carrier, adjuvant and/or excipient.

As used in the description, the expression "therapeutically effective amount" refers to the amount of Ca10 inhibitor calculated to produce the required effect and, in general, will be determined, among other factors, by the inherent properties of the compounds and the subjects to be treated, including age, patient's condition, severity of alteration or disorder, and the route and frequency of administration. The methods and tools for calculating the therapeutically effective amount of the Ca10 inhibitor are common general knowledge for the skilled person in the art.

The pharmaceutically acceptable adjuvants, excipients and carriers that may be used in said compositions are the adjuvants, excipients and carriers known to those skilled in the art and commonly used to produce therapeutic compositions. The term "pharmaceutically acceptable carrier" is a substance used in the composition to dilute any of the components comprised therein to a certain volume or weight. The pharmaceutically acceptable carrier is an inert substance or having action identical to any of the elements comprised in the composition of the present invention. The function of the carrier is to facilitate the incorporation of other elements, allow better dosage and administration or provide consistency and shape to the composition. Additionally, the pharmaceutical composition of the invention may comprise one or more adjuvants and/or excipients. The term "pharmaceutically acceptable excipient" refers to a substance that assists in the absorption of the elements of the composition of the invention, stabilises said elements, activates or assists in the preparation of the composition in the sense of providing consistency or providing flavours that make it more pleasant. Therefore, the excipients could have the function of keeping the ingredients linked together, such as for example in the case of starches, sugars or cellulose, the function of sweetening, the function of colouring, the function of protecting the composition, such as for example isolating it from air and/or moisture, the function of filling a tablet, capsule or any other form of presentation, a disintegrating function to facilitate the dissolution of the components and the absorption thereof in the intestine, without excluding other types of excipients not mentioned in this paragraph.

Examples of pharmaceutically acceptable carriers are known in the art and include phosphate-buffered saline solutions, water, emulsions, such as oil/water emulsions, different types of wetting agents, sterile solutions, etc. The compositions comprising said carriers can be formulated by conventional procedures known in the state of the art. As used in the present specification, the terms "pharmaceutically acceptable", "physiologically tolerable" and grammatical variations thereof, when referring to compositions, supports, diluents and reagents, are used interchangeably and indicate that the materials can be administered to a subject without producing undesirable physiological effects.

The pharmaceutical composition is prepared in solid form or in aqueous suspension, in a pharmaceutically acceptable diluent. The composition provided by the present invention can be administered via any suitable route of administration, such that said composition will be formulated in the suitable pharmaceutical form for the selected route of administration. In a particular embodiment, the administration of the composition provided by the invention is carried out parenterally, orally, intraperitoneally, subcutaneously, intracranially, etc. The composition for use thereof as described herein may further comprise other drugs or agents, preferably chemotherapeutic, used in the treatment of cancer with a view to acting in a complementary or reinforcing manner. In another even more particular embodiment, chemotherapeutic agents are selected from the list consisting of: cisplatin, carboplatin, docetaxel, gemcitabine, paclitaxel, vinorelbine, etoposide, vinblastine, pemetrexed and irinotecan.

The Ca10 inhibitor of the present inventor can be used for treating cancer.

As used herein, the term "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g. preventing or delaying the worsening of the disease), preventing or delaying the spread (e.g. metastasis) of the disease, preventing or delaying the recurrence of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. In the context of the present invention, the inhibitory agent of the invention inhibits or reduces relapse, growth, progression, worsening or metastasis of the tumor or cancer; results in partial or complete destruction of the neoplastic, tumor, cancer or malignant cell mass, volume, size or numbers of cells, stimulating, inducing or increasing neoplastic, tumor, cancer or malignant cell necrosis, lysis or apoptosis, reducing neoplasia, tumor, cancer or malignancy volume size, cell mass, inhibiting or preventing progression or an increase in neoplasia, tumor, cancer or malignancy volume, mass, size or cell numbers, or prolonging lifespan; results in reducing or decreasing severity, duration or frequency of an adverse symptom or complication associated with or caused by the neoplasia, tumor, cancer or malignancy; or method results in reducing or decreasing pain, discomfort, nausea, weakness or lethargy, or results in increased energy, appetite, improved mobility or psychological wellbeing.

In a particular embodiment of the Ca10 inhibitor, or the composition or kit comprising it, for use according to the present invention, the cancer is a carcinoma or an adenocarcinoma.

In another particular embodiment of the Ca10 inhibitor, or the composition or kit comprising it, for use according to the present invention, the cancer is prostate cancer, cervix cancer, breast cancer, pancreatic cancer, lung cancer, gastric cancer, endometrial cancer, skin cancer, thyroid cancer, bladder cancer, ovarian cancer, lever cancer, esophageal cancer, or colorectal cancer.

In another particular embodiment of the Ca10 inhibitor, or the composition or kit comprising it, for use according to the present invention, the monoclonal antibody is administered in combination with another cancer immunotherapy, in particular, with one or more other drugs in the treatment, control, amelioration, or reduction of cancer, where the combination of the drugs together are safer or more effective than either drug alone. Examples of drugs which may be used in combination with the Ca10 inhibitor of the invention for treatment, prevention, control, amelioration, or reduction of cancer include, without limiting to, cisplatin or carboplatin, gemcitabine, paclitaxel, docetaxel, etoposide and vinorelbine.

### DESCRIPTION OF THE DRAWINGS

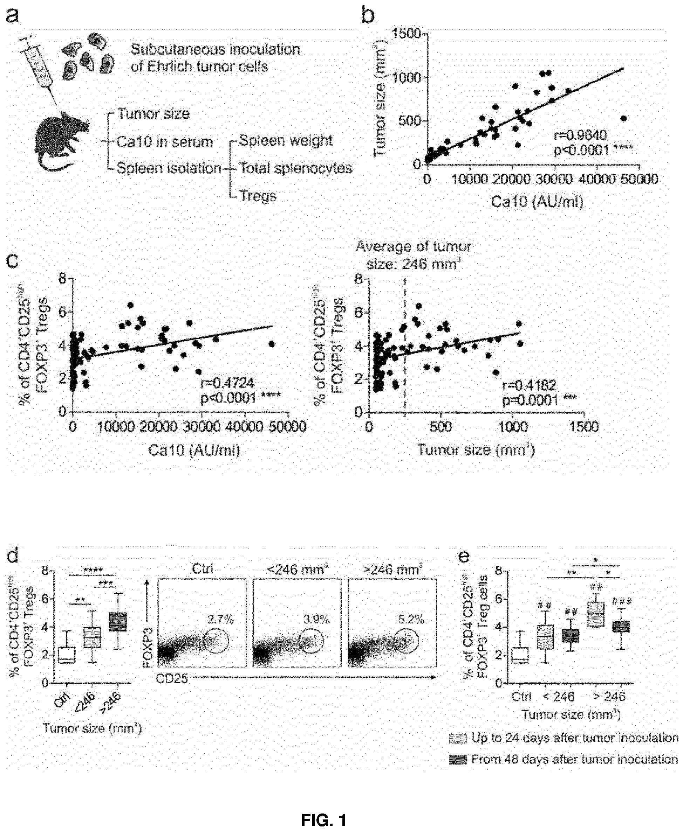

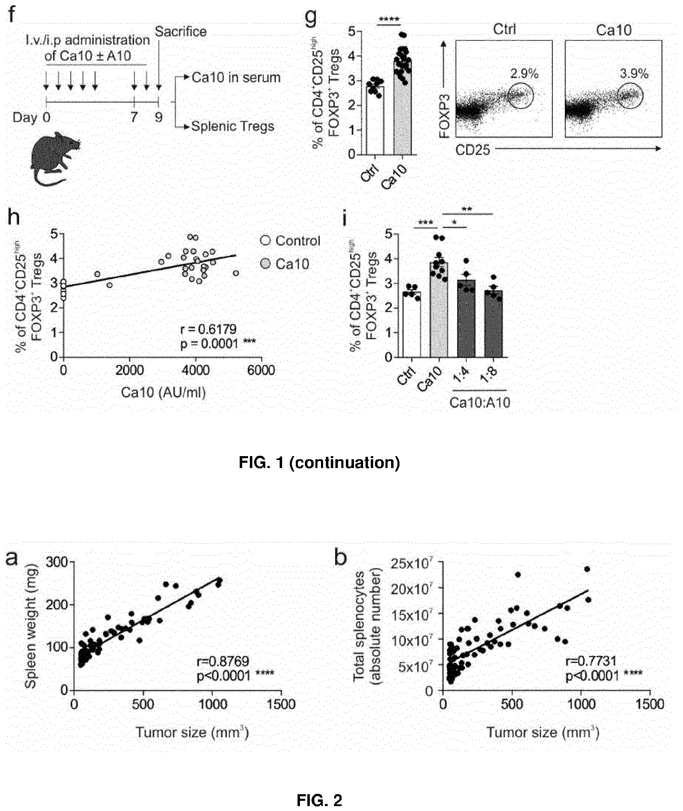

**Figure 1****. a,** Subcutaneous inoculation protocol with ET cells in mice. **B-c,** Correlation of serum Ca10 levels with tumor size (mm³) **(b)** and percentage of splenic Tregs with serum Ca10 levels or tumor size (mm³) **(c)** in ET-bearing mice (n = 79 mice of two independent experiments). **D,** Percentage of splenic Tregs from no tumor-bearing mice (ctrl) and mice with small (< 246 mm³) or large (> 246 mm³) tumors. The average tumor size was used as cut off (246 mm³). Representative dot plots of Tregs are shown (n = 6 for ctrl, n = 48 and 25 for small and large tumors, respectively, of two independent experiments). **E,** Percentage of splenic CD4+CD25^{high}FOXP3+ Tregs from no tumor-bearing mice (ctrl) and small or large tumor-bearing mice. Mice were sacrificed up to 24 days after tumor inoculation (light grey) or from 48 days after tumor inoculation (dark grey) (n = 6 mice for ctrl, n = 30 mice with small tumor sacrificed up to 24 days after ET inoculation, n = 18 mice with small tumor sacrificed from 48 days after ET inoculation, n = 6 mice with large tumor sacrificed up to 24 days after ET inoculation, and n = 19 mice with large tumor sacrificed from 48 days after ET inoculation of two independent experiments). # tumor-bearing mice vs control, * tumor condition vs tumor condition. **F,** Scheme of the alternative intravenous (i.v.) and intraperitoneal (i.p.) administration protocol of Ca10 in the presence or absence of the mAb A10 in mice. **G,** Percentage of splenic CD4+CD25^{high}FOXP3+ Tregs from control mice and mice treated with Ca10. Representative dot plots of generated Tregs are shown (n = 9 mice for ctrl and n = 24 mice for Ca10, of two independent experiments). **H,** Correlation of percentage of splenic Tregs with serum Ca10 levels in control mice (n = 9) and Ca10-administered mice (n = 24 mice of two independent experiments). **I,** Percentage of splenic CD4+CD25^{high}FOXP3+ Tregs from control mice and mice treated with Ca10 in the absence or presence of the mAb A10 (n = 5 mice for ctrl, n = 10 mice for Ca10 and n = 4 mice for Ca10 in the presence of mAb A10 at ratios 1:4 or 1:8, of one independent experiment). Values are mean ± SEM. Statistical significance was determined using Spearman test (b, c and h), Mann-Whitney test (d and e) and Unpaired Student t test (g and i). **P <* 0.05, ***P <* 0.01, ****P* < 0.001, and *****P* < 0.0001.
**Figure 2****. a,** Correlation of spleen weight with tumor size from ET-bearing mice. **B,** Correlation of total number of cells in spleen of ET-bearing mice with tumor size. Values are mean ± SEM of 79 individual mice of individual mice of two independent experiments. Statistical significance was determined using Spearman test. *****P* < 0.0001.

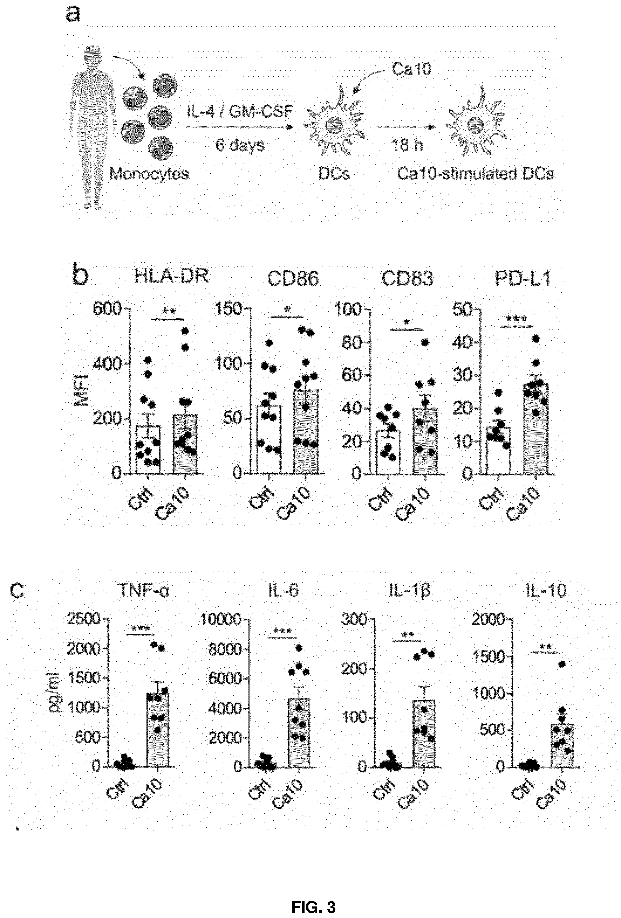

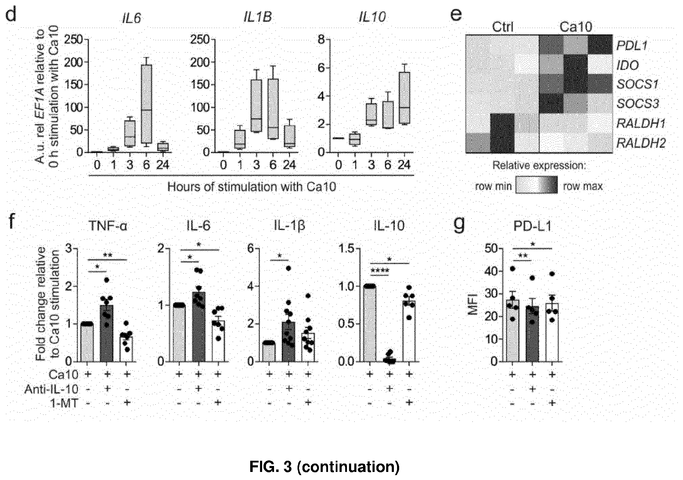

**Figure 3****. a,** Human *in vitro* model for the generation of human monocyte-derived DCs. **b,** Mean fluorescence intensity (MFI) values for the surface markers HLA-DR, CD86, CD83 and PD-L1 after stimulation of human DCs with medium (Ctrl) or Ca10 for 18 h (n = 8-10). **C,** Cytokine production after stimulation of human DCs with medium or Ca10 for 18 h quantified in cell-free supernatants by ELISA (n = 8-9). **d,** Messenger RNA expression levels of *IL6, IL1B* and *IL10* genes in human DCs treated with Ca10 for 0, 1, 3, 6 and 24 h relative to Ca10 stimulation at 0 h. Arbitrary units (A.u.) are 2^{-ΔCT} values multiplied by 10⁴, with ΔCT defined as the difference between the cycle threshold value for the gene of Interest and *EF1A* (n = 4). **e,** Heatmap representation for gene expression on human DCs unstimulated or stimulated with Ca10 for 24 h (n = 3). **f,** Cytokine production of human DCs stimulated with Ca10 in the presence of IL-10 blocking antibody or IDO inhibitor (1-MT) for 18 h relative to Ca10 stimulation (n = 6-10). **g,** MFI values for the expression of surface marker PD-L1 on human DCs after stimulation with Ca10 in the presence of IL-10 blocking antibody or 1-MT for 18 h (n = 5). Values are mean ± SEM. Statistical significance was determined using Paired Student t test. **P* < 0.05, ***P <* 0.01, ****P* < 0.001, and *****P* < 0.0001.

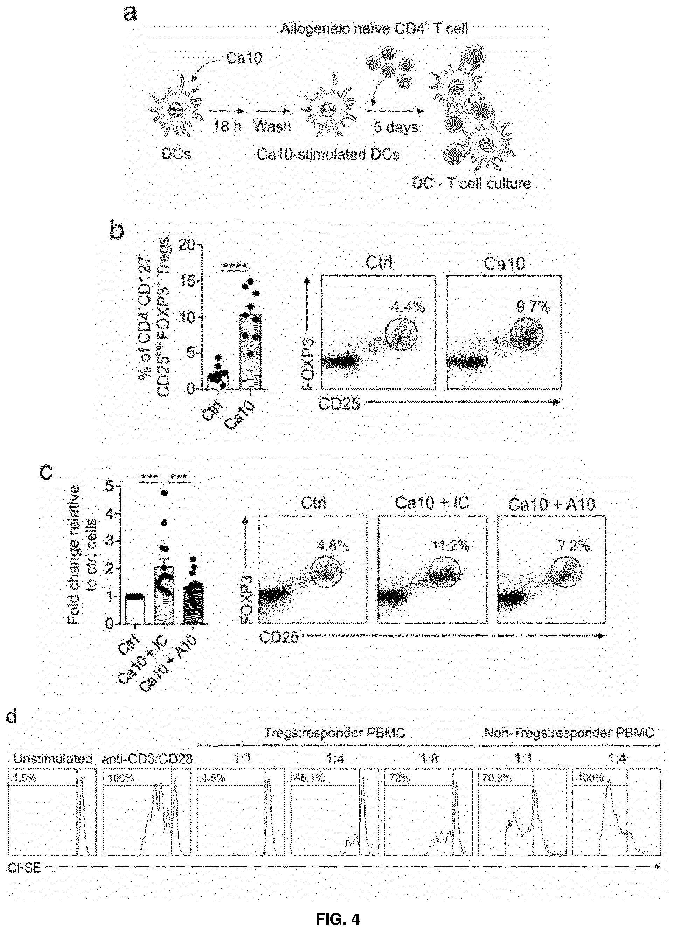

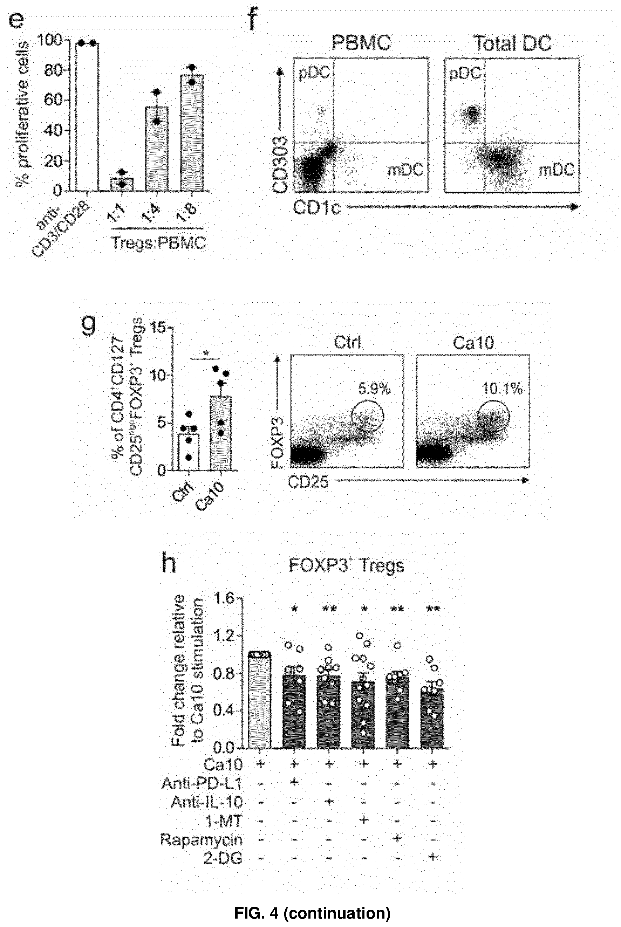

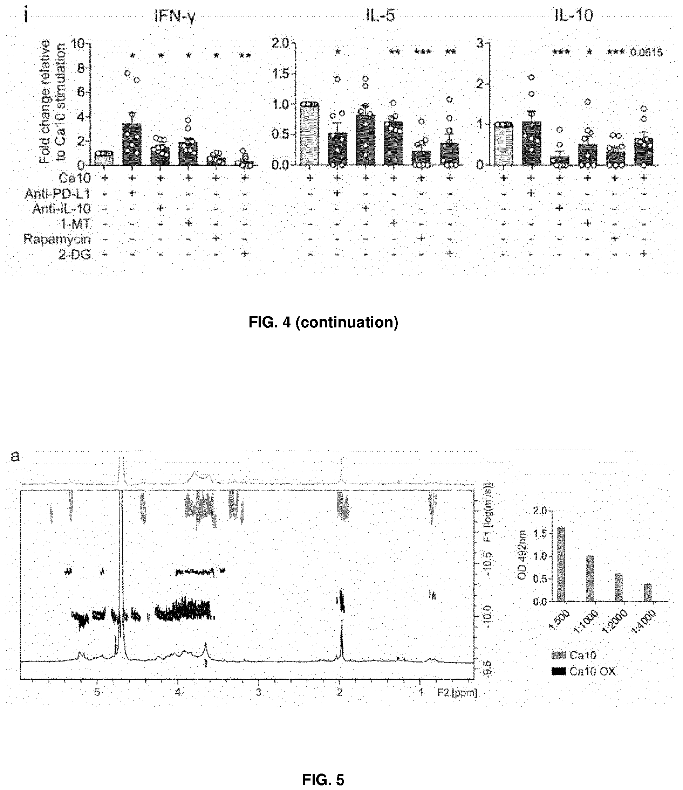

**Figure 4****. a,** Human *in vitro* model for allogeneic coculture. **b,** Percentage of Tregs gated on CD4⁺ T cells after 5 days of coculture with unstimulated or Ca10-treated human DCs (n = 9). Representative dot plots are shown, **c,** Fold change of Tregs gated on CD4+ T cells generated by DCs treated with Ca10 in the presence of the mAb A10 (A10) or the corresponding isotype control (IC) after 5 days relative to control DCs (pool of 1:10 and 1:5 ratios, Ca10:A10) (n = 14). **d,** Suppression assays of purified Tregs and non-Tregs induced by Ca10-activated DCs. **e,** Percentage of proliferating CD4+ cells after 5 days of stimulation in the presence of autologous purified Tregs induced by Ca10-activated DCs at different ratios (n = 2). **f,** Representative dot plots of different DCs subsets in PBMCs and total DCs fraction gating on HLA-DR⁺CD19⁻ cells. Total DCs fraction containing both HLA-DR⁺CD1c⁺ myeloid and HLA-DR⁺CD303⁺ plasmacytoid DCs are shown. **g,** Percentage of Tregs by untreated (ctrl) or Ca10-treated total blood DCs after 5 days (n = 5). Representative dot plots are shown. **h,** Percentage of Tregs induced by Ca10-treated DCs in the presence of blocking antibodies of PD-L1 or IL-10, or inhibitors for IDO (1-MT), mTOR (rapamycin) or glycolysis (2-DG) after 5 days (n = 8-12) relative to Ca10-stimulation alone. **i,** Cytokines produced by allogeneic naive CD4+ T cells primed by Ca10-activated DCs after 5 days under the indicated conditions relative to Ca10-stimulation alone (n = 8-12). Values are mean ± SEM. Statistical significance was determined using Paired Student *t* test (b, g, h and i) and Wilcoxon test I. **P* < 0.05, ***P* < 0.01, ****P* < 0.001, and *****P* < 0.0001.

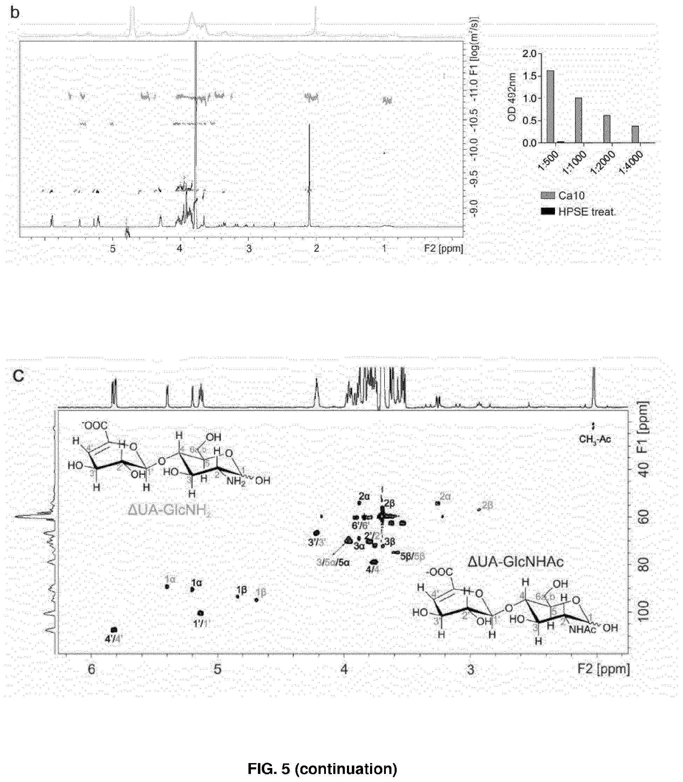

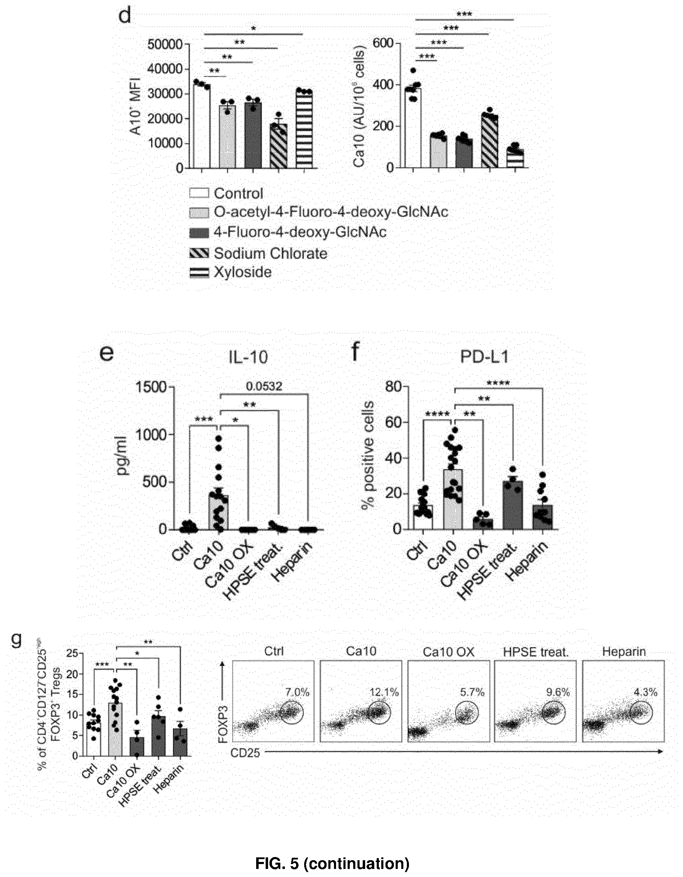

**Figure 5****. a,** Two dimensional (2D) Diffusion Ordered Spectroscopy DOSY and one dimensional, 1D-1H spectra of Ca10 (Control, grey) and Ca10 subjected to oxidation with NaIO₄ (Ca10 OX, black). 2D-DOSY spectrum of pullulan 100 kDa is included as size standard; all of them acquired a 298 K; vertical axis corresponds to logarithm of diffusion coefficient. Inset, A10 mAb activity before (Ca10) and after oxidation (Ca10 OX) determined by ELISA. **b,** 2D-DOSY and 1D-1H spectra of Ca10 (control, grey), Ca10 subjected to degradation treatment with heparinase (HPSE treat., black). 2D-DOSY spectra of Pullulan 100 kDa (black) and ΔUA-GlcNAc, 0,38 kDa are included as size standards; all of them acquired a 303 K. Inset, A10 activity before (Ca10) and after treatment with HPSE (HPSE treat.) determined by ELISA. **c,** Heteronuclear single quantum coherence spectra (HSQC) spectra of heparinase-digested Ca10 disaccharides with corresponding peak assignments (unlabelled signals correspond to buffer components tris and glycerol). **d,** Mean fluorescence intensity (MFI) for A10⁺ ET cells and Ca10 concentration in supernatants after 24 h (AU/10⁶ ET cells) in the presence of glycosaminoglycan biosynthesis inhibitors O-acetyl-4-fluoro-4-deoxy-GIcNAc, 4-fluoro-4-deoxy-GIcNAc, Sodium chlorate and Xyloside (n = 3-7). **e,** IL-10 production and **f,** percentage of PD-L1+ human DCs unstimulated or stimulated with Ca10, Ca10 OX, Ca10 HPSE treat. or in the presence of heparin for 18 h (n = 4-10). **g,** Tregs induced after 5 days of priming by control-, Ca10-, Ca10 OX- or Ca10 HPSE treat.-stimulated human DCs or Ca10-treated human DCs in the presence of heparin (n = 4-6). Values are mean ± SEM. Statistical significance was determined using Unpaired Student t test (d) and Paired Student t test (e-g). **P* < 0.05, ***P* < 0.01, and ****P* < 0.001.
**Figure 6****. a-b,** HSQC spectra of heparan sulfate disaccharide standards ΔUA-GIcN **(a)** and ΔUA-GIcNAc **(b)** with the corresponding peak assignments.
**Figure 7****. a,** Percentage of Tregs gated on CD4⁺ T cells after 5 days of coculture with unstimulated, Ca10- or Ca10+pronase-treated human DCs (n = 4). Representative dot plots are shown. **b,** A10 mAb activity before (Ca10) and after pronase treatment (Ca10+pronase) determined by ELISA (n = 4). Values are mean ± SEM. Statistical significance was determined using Paired Student t test (a). ***P* < 0.01.
**Figure 8****.** Regression line used to calculate serum circulating Ca10 levels (Ca10H) in human sera.
**Figure 9****. a,** Scheme of the protocol to determine human Ca10 homologue (Ca10H) concentrations in serum from healthy donors and cancer patients. **b,** Concentrations of Ca10H in serum from healthy controls (n=131), and patients diagnosed with prostate cancer (n=248), colorectal cancer (n=66) or other types of cancer (n=71). **c,** Concentrations of Ca10H in serum from prostate cancer patients with (n=42) or without (n=30) metastases. **d,** Correlations between Ca10H and alkaline phosphatase serum levels in prostate cancer patients with or without metastases. **e,** Concentrations of Ca10H in untreated, HPSE or Sialidase treated serum from different prostate cancer patients (n=3). Values are mean ± SEM. Statistical significance was determined using Mann-Whitney test (b and c), Spearman test (d) and Unpaired Student *t* testI). ***P* < 0.01, and ****P* < 0.001.
**Figure 10****.** Serum levels of Ca10H in healthy control subjects and in patients with various types of cancer at any clinical stage. Values are mean ± SEM.
**Figure 11****.** Serum levels of Ca10H in healthy control subjects and in patients with prostate cancer (PCa) or benign prostatic hyperplasia (BPH) at any clinical stage. Values are mean ± SEM. Statistical significance was determined using Mann-Whitney test.
**Figure 12****.** Correlation between the serum levels of Ca10H and PSA (prostate-specific antigen) in patients with prostate cancer at any clinical stage. Correlation was determined using Spearman rank correlation coefficient.
**Figure 13****.** Serum levels of Ca10H in healthy control subjects and in patients with fibroadenoma (FA) or breast cancer at any clinical stage. Values are mean ± SEM. Statistical significance was determined using Mann-Whitney test.
**Figure 14****.** Correlation between the serum levels of Ca10H and CA 15.3 (A) or CEA (Carcinoembryonic antigen) (B) in patients with breast cancer at any clinical stage. Correlation was determined using Spearman rank correlation coefficient.
**Figure 15****.** Serum levels of Ca10H in healthy control subjects and in patients with colon polyps or colorectal cancer (Colon Ca) at any clinical stage. Values are mean ± SEM. Statistical significance was determined using Mann-Whitney test.
**Figure 16****.** Correlation between the serum levels of Ca10H and CA 19.9 (A) or CEA (Carcinoembryonic antigen) (B) in patients with colorectal cancer at any clinical stage. Correlation was determined using Spearman rank correlation coefficient.

### Examples

### Example 1. Serum Ca10 levels correlate positively with the frequency of splenic Tregs in Ehrlich tumor-bearing and tumor-free mice.

### Material and Methods

Ehrlich tumor (ET) cells, initially originated from hyperdiploid Ehrlich-Lettré mouse ascite tumor cells, are derived from a cloned cell variant selected for its high reactivity with the monoclonal antibody A10.

The monoclonal antibody A10 (Gil, J. et al.1990. Cancer Res 50, 7301-7306) was purified from the hybridoma culture supernatant as follows:
BALB/c mice were immunized with mitomycin-treated ET cells (Subiza, J. L., Coll, J., Alvarez, R., Valdivieso, M., and De la Concha, E. G. Cancer Immunol. Immunother., 25: 87-92. 1987). Cell fusion was carried out with spleen cells from an immunized mouse (3 days after the last dose), following the method of Köhler and Milstein (Köhler,G., and Milstein, C. Nature (Lond.). 256:495-497, 1975.) with minor modifications. Briefly, mouse spleen cells (5 × 10⁷) were fused with FO (nonsecreting myeloma) cells (2 × 10⁷) in polyethylene glycol (PEG 4000; Merck, Darmstadt, Federal Republic of Germany). The fused cells were distributed into 96-well microtiter plates and cultured in hypoxanthine-azaserine-RPMI at 37ºC in 5% CO₂ incubator. Hybridoma culture supernatants were assayed for anti-ET cell surface carbohydrates by ELISA as described below. Cultures showing IgM reactivity towards these carbohydrates were cloned twice by limiting dilution using thymocytes as feeder layers, picking up only from those wells where a single clone could be assured microscopically. Stable cultures of antibody-producing hybridomas were expanded and monoclonal antibodies produced in pristane-trated BALB/c mice. Cell-free ascites fluid from each hybridoma was recovered in sterile conditions, filtered (0.45 µm; Millipore, Molsheim, France), decomplemented (40 minutes at 56°C) and stored at -40°C in aliquots until used

The monoclonal antibody JM403 (commercialised by AMSBIO) which specifically recognize the saccharide portion of the Ca 10, i.e.: deacetylated glucosamine residues of heparosan as shown in the formula disclosed in the present description, may also be used in the context of the present invention.

Ca10 was obtained from 24 h supernatants of ET cells growing *in vitro* in serum-free medium. Briefly, the pooled supernatants collected by centrifugation were subjected to tangential ultrafiltration on 300 kDa membranes under high ionic strength (1 M NaCl). The Ca10-enriched fraction recovered in the retentate, was subsequently diafiltrated against distilled water on 300 kDa membranes and lyophilized, having a relative composition of 85±5% carbohydrate and 10±5% protein with no significant amounts of nucleic acids or lipids.

C57BL/6J mice were inoculated with 10⁵ ET cells by subcutaneous injection into the left groin. Tumor size was monitored weekly by calculating the tumor volume measured with a Vernier caliper. Mice were sacrificed at different time points (days) of tumor development to measure Ca10 in sera (ELISA) and Tregs in spleen (flow cytometry). In some experiments, tumor-free mice were administered (i.v./i.p.) with isolated Ca10 (µg) daily for 7 days with or without different amounts of mAb A10 (80, 160 or 320 µg)

### Results

Ehrlich tumor (ET) is a paradigmatic example of tumor progression across different antigenic backgrounds, representing a suitable model to unravel tumor escape mechanisms. ET-bearing mice show increasing amounts of Ca10 in their sera as the tumor develops. To assess the potential relationship between circulating Ca10 and Tregs generation in their spleen, we used solid ET-bearing mice **(****Figure 1a****).** A strong positive correlation was observed between serum Ca10 levels and tumor size was confirmed **(****Figure 1b****).** The percentage of splenic FOXP3⁺ Tregs showed also a moderated but significant positive correlation with both serum Ca10 levels and tumor size **(****Figure 1c****).** Consistent with previous studies, a strong splenomegaly related to tumor size was observed concomitant with a positive correlation between total number of splenocytes and tumor size **(****Figures 2a, 2b****).** The variability of tumor size among the different ET-bearing mice allowed the setting of a cut-off point of 246 mm³ (the average tumor size) to stratify mice bearing large (>246 mm³) and small (<246 mm³) tumors. The percentage of splenic FOXP3⁺ Tregs was significantly higher in mice bearing large than small tumors, regardless of the time elapsed since tumor inoculation, and always higher than controls **(****Figure 1d****).** The percentage of splenic Tregs in mice bearing large tumors (>246 mm³) that were developed at earlier time points (up to 24 days after tumor inoculation) was significantly higher than in large tumors reaching that size at later time points (from 48 days onwards after inoculation of ET cells) **(****Figure 1e****).** These data suggest a close relationship between serum Ca10 levels, the induction of splenic Tregs, and tumor progression. To further investigate whether Ca10 might be directly involved in the observed increase of Tregs, isolated Ca10 from ET cell culture supernatants was administered to C57BL/6J tumor-free mice either alone or in combination with the mAb A10 **(****Figure 1f****).** Remarkably, the percentage of splenic FOXP3⁺ Tregs was significantly higher in mice administered with Ca10 than control mice, which was observed just after 9 days of Ca10 administration **(****Figure 1g****).** Of note, in these experiments a significant positive correlation between serum Ca10 levels and the percentage of splenic Tregs was observed **(****Figure 1h****).** The administration of Ca10 in combination with the Ca10-reacting mAb A10 significantly neutralized the generation of splenic Tregs induced by Ca10 in a dose-dependent manner **(****Figure 1i****).** This indicates that mAb A10 completely blocks 'a10's capacity to induce splenic Tregs. Thus, Ca10 released from the tumor appears to be involved in the generation of Tregs in the ET-bearing mice, which in turn may promote tumor progression.

### Example 2. Ca10 endorses tolerogenic features in human monocyte-derived dendritic cells (hmoDCs) that generate functional Tregs in vitro

### Material and Methods

Peripheral blood mononuclear cells (PBMC) from buffy coats of healthy donors were obtained by using Ficoll density gradient centrifugation. Monocytes were isolated from total PBMC using anti-CD14 microbeads and cultured for 6 days with RPMI medium containing 100 ng/mL of IL-4 and GM-CSF to generate immature human monocyte-derived dendritic cells (hmoDCs). To generate Ca10/hmoDCs, Ca10 (20 µg/mL) was added on days 0 and 4 of the differentiation. Peripheral blood naive CD4+ T cells and total DCs fraction were isolated using commercially available kits. In all cases, the purity and phenotype of cells were analyzed by flow cytometry with lineage-specific markers, and cell viability was determined by trypan blue exclusion.

Immature hmoDCs (10⁶ cells per mL) were treated with Ca10 (20 µg/mL) for 18 h. Subsequently, cells were collected and centrifuged. Cell pellets were used to analyze their phenotype by flow cytometry and qPCR, and cell-free supernatants were used to quantify TNF-α, IL-6, IL-1β and IL-10 by ELISA. For inhibition experiments, hmoDCs were preincubated for 30 min with EDTA (0.5 mM), piceatannol (25 µM), U0126 (1 µM), SB202190 (1 µM), SP600125 (10 µM), BAY117082 (2.5 µM), 1-MT (1 mM), Rapamycin (100 nM), or heparin (100 IU/ml); and for 1 h with 2-DG (10 mM) or anti-IL-10 (2.5 µg/ml), or corresponding vehicle control or isotype control prior to treatment. Then, hmoDCs were treated with Ca10 for 18 h in the presence of the corresponding inhibitors. For blocking experiments with the mAb A10, Ca10 was previously incubated during 1 h with mAb A10, or corresponding isotype control, in a ratio of 1:10 or 1:5 (Ca10:A10) with continuous stirring, and then, it was added to hmoDCs for 18 h.

RNA was isolated from harvested cells using an RNeasy and cDNA generated using a PrimeScript RT reagent Kit. Real-time quantitative PCR was performed on cDNA using FastStart Universal SYBR Green Master. The sequences of the used pair primers are shown in Table 1.

**Table 1 - Reverse and Forward primer used in the present invention. IDO: indoleamine 2,3-dioxygenase; SOCS1: suppressor of cytokine signaling 1; SOCS3: suppressor of cytokine signaling 3; RALDH1: retinaldehyde dehydrogenase 1; RALDH2: retinaldehyde dehydrogenase 2; EF1A: Elongation Factor 1α.**

| Gene | Primer | Nucleotide sequence |
|---|---|---|
| *IL6* | Forward | GGTACATCCTCGACGGCATCT (SEQ ID NO: 1) |
| | Reverse | GTGCCTCTTTGCTGCTTTCAC (SEQ ID NO: 2) |
| *IL1B* | Forward | TTTTTGCTGTGAGTCCCGGAG (SEQ ID NO: 3) |
| | Reverse | TTCGACACATGGGATAACGAGG (SEQ ID NO: 4) |
| *IL10* | Forward | GTGATGCCCCAAGCTGAGA (SEQ ID NO: 5) |
| | Reverse | CACGGCCTTGCTCTTGTTTT (SEQ ID NO: 6) |
| *PDL1* | Forward | AAGATGAGGATATTTGCTGTCTTTATATTC (SEQ ID NO: 7) |
| | Reverse | GTCCTTGGGAACCGTGACAGT (SEQ ID NO: 8) |
| *IDO* | Forward | AGAAGTGGGCTTTGCTCTGC (SEQ ID NO: 9) |
| | Reverse | TGGCAAGACCTTACGGACATCTC (SEQ ID NO: 10) |
| *SOCS1* | Forward | CCCTGGTTGTTGTAGCAGCTT (SEQ ID NO: 11) |
| | Reverse | CAACCCCTGGTTTGTGCAA (SEQ ID NO: 12) |
| *SOCS3* | Forward | CCTCAGCATCTCTGTCGGAAGA (SEQ ID NO: 13) |
| | Reverse | GCATCGTACTGGTCCAGGAACT (SEQ ID NO: 14) |
| *RALDH1* | Forward | CTGCCGGGAAAAGCAATCT (SEQ ID NO: 15) |
| | Reverse | AAATTCAACAGCATTGTCCAAGTC (SEQ ID NO: 16) |
| *RALDH2* | Forward | AGGGCAGTTCTTGCAACCAT (SEQ ID NO: 17) |
| | Reverse | GCGTAATATCGAAAGGTTTTGATGA (SEQ ID NO: 18) |
| *EF1A* | Forward | CTGAACCATCCAGGCCAAAT (SEQ ID NO: 19) |
| | Reverse | GCCGTGTGGCAATCCAAT (SEQ ID NO: 20) |

Samples were run on a real-time PCR system. Data were normalized to *EF1A* and displayed as arbitrary units calculated as 2^{-ΔCT} values multiplied by 10⁴. ΔCT was defined as the difference between the cycle threshold value for the gene of interest and *EF1A.*

### Results

To further explore the mechanisms involved in the potential capacity of Ca10 to promote Tregs generation, we initially investigated the ability of this tumor-associated carbohydrate to regulate the phenotype and function of human DCs. Ca10 stimulation of DCs differentiated from human blood monocytes of healthy donors **(****Figure 3a****)** significantly increased the expression of HLA-DR, CD86/CD83 and the tolerogenic marker PD-L1 **(****Figure 3b****).** Ca10-stimulated DCs also produced significantly higher levels of TNF-α, IL-6, IL-1β as well as the anti-inflammatory cytokine IL-10 than unstimulated cells **(****Figure 3c****).** Kinetic experiments showed that Ca10 rapidly increased the mRNA expression of pro-inflammatory cytokines in a time-dependent manner, which declined to almost basal levels after 24 h of stimulation **(****Figure 3d****).** In contrast, the mRNA levels of IL-10 **(****Figure 3d****)** and the tolerogenic molecules PD-L1, IDO, SOCS1 and SOCS3 **(****Figure 3e****)** remained upregulated after 24 h of stimulation, suggesting that Ca10 might well imprint tolerogenic features in human DCs. Supporting these data, blocking of IL-10 significantly enhanced the production of the pro-inflammatory cytokines TNF-α, IL-6 and IL-1β whereas treatment with 1-methyl tryptophan (1-MT), a selective inhibitor of IDO activity, significantly reduced the production of IL-10 in Ca10-stimulated DCs **(****Figure 3f****).** Blocking of IL-10 or IDO also reduced the induction of PD-L1 in Ca10-stimulated DCs **(****Figure 3g****),** indicating that the tolerogenic features imprinted by Ca10 in human DCs are partially depending on IL-10 and IDO induction.

To determine whether Ca10 could indeed generate human tolerogenic DCs with the capacity of polarize functional Tregs, we performed coculture experiments with allogeneic naive CD4+ T cells **(****Figure 4a****).** Ca10-stimulated human DCs induced significantly higher percentage of CD4+CD127-CD25^{high}FOXP3+ Tregs than unstimulated control DCs **(****Figure 4b****).** When Ca10 was previously incubated with the blocking mAb A10, the frequency of Tregs induced by Ca10-activated DCs was significantly decreased **(****Figure 4c****).** Remarkably, the induced Tregs by Ca10-stimulated DCs were functional as they were able to potently suppress, in contrast to the non-Tregs fraction, the proliferation of autologous PBMC in a dose-dependent manner **(****Figure 4d****,** **4e****).** Supporting these findings, an enriched fraction of human blood total DCs containing both plasmacytoid (pDC) and myeloid (mDC) DCs **(****Figure 4f****)** stimulated with Ca10 also generated significantly higher percentage of CD4+CD127-CD25^{high}FOXP3⁺ Tregs than unstimulated total DCs **(****Figure 4g****).** Blocking of PD-L1, IL-10, IDO, mTOR and glycolysis significantly impairs the generation of Tregs by Ca10-stimulated DCs **(****Figure 4h****),** which was accompanied by significantly lower levels of IL-5 and IL-10 **(****Figure 4i****).** Blocking of PD-L1, IL-10 and IDO induced significantly higher levels of IFN-y (especially after PD-L1 inhibition), whereas inhibition of mTOR and glycolysis significantly reduced the production of IFN-γ **(****Figure 4i****).**

### Example 3. Murine Ehrlich tumor-derived Ca10 is a heparan sulfate-related glycosaminoglycan.

### Material and Methods

NMR spectra were acquired using a Bruker AVANCE 600 MHz spectrometer equipped with a triple resonance TXI cryogenic probe and processed with TOPSIN 3.0 software (Bruker SA). NMR samples were prepared in deuterium oxide (D₂O). One dimensional proton (1D-¹H) spectra and two-dimensional (2D) Diffusion-*O*rdered Spectroscop*y* (DOSY), 2D Heteronuclear ¹H-¹³C *S*ingle *Q*uantum *C*oherence (¹H-¹³C HSQC) and 2D homonuclear ¹H-¹H Total *C*orrelation *S*pectroscop *Y* (TOCSY) spectra were acquired using standard pulse sequences included in TOPSPIN software to characterize the structure (TOCSY and HSQC) and hydrodynamic behaviour (DOSY) of Ca10 samples.

To monitor periodate oxidation, Ca10 was dissolved in 50 mM sodium periodate (NaIO₄) in D₂O at a final concentration of 5 mg/mL in an NMR tube and was introduced in the spectrometer probe adjusted at 298 K. One dimensional 1D-¹H and 2D DOSY spectra were acquired at sequential times to follow the oxidation reaction during 8 h. DOSY spectra were performed using the standard Bruker pulse sequence (ledbpgp2s), acquiring 16 gradient points, with 128 scans each, between 2 and 95% gradient intensity using a diffusion time delay of 0.25 s and 2500 µs wide pulse gradient.

To monitor heparinase digestion, 5 µL of Bacteroides heparinases II and III (400 UA/mL and 700 UA/mL, respectively) were added to the NMR tube containing 5 mg/mL Ca10 sample in heparinase buffer and the tube was introduced in the spectrometer probe adjusted at 303 K. To follow the enzymatic reaction, 1D-¹H and 2D DOSY spectra were acquired at sequential times during 24 h. The 1D-¹H spectra of 128 scans of 32 K size were recorded applying the TOPSIN zgesgp pulse program that includes a gradient sculpting water suppression procedure. The 2D DOSY experiments were performed using the pulse sequence ledbpgp2s acquiring 32 gradient points, with 128 scans each between 2 and 95% gradient intensity. The diffusion time delay and gradient duration were 600 ms and 2500 µs, respectively, before the starting of the reaction and 170 ms and 1700 µs at end point of the reaction. Enzyme digestions. A collection of glycosidases was obtained; N-glycosidase (PNGase F), O-glycanase (endo-galactosaminidase), endo-β-acetylglucosaminidase, exoglycosidases (α- and β- galactosidases), α-mannosidase, glucosidases, glucosaminidase, α-fucosidase, sialidase (NZYTECH), chitosanase 8B, heparinases (New England Biolabs) and Chondroitinase ABC. Working conditions with each enzyme have been set up using model glycoproteins such as α1-acid glycoprotein, fetuin, asialofetuin, ribonuclease B and commercial oligosaccharides. Enzymatic digestions were analysed by NMR, Polyacrylamide gel electrophoresis (SDS-PAGE) and Ca10-epitope sandwich ELISA evaluation.

The sample of heparinases-digested Ca10 was filtered using Vivaspin^{™} 50K Centrifugal Concentrators (Sartorius), and the filtrate was lyophilized and resuspended in D₂O for NMR analysis. 1D-¹H, 2D TOCSY, ¹H-¹³C HSQC and DOSY spectra were acquired. For comparison a collection of heparan sulfate disaccharide standards were also NMR analyzed: ΔUA,2S - GIcNAc,6S (I-A) ΔUA - GlcNS,6S (II-S), ΔUA - GIcNS (IV-S), ΔUA - GIcNAc (IV-A), ΔUA,2S - GIcNAc (III-A) (Iduron, UK) and ΔUA - GlcN (IV-H) (Santa Cruz Biotechnology). Samples of each of them at 1 mM concentration in D₂O were prepared and 1D-¹H, 2D ¹H-¹³C HSQC and DOSY spectra acquired for comparison.

To analyse the contribution of the carbohydrate structure, hmoDCs were stimulated with 20 µg/mL of Ca10 (without treatment), oxidized Ca10 (Ca10 OX), Ca10 treated with Heparinase (HPSE treat.) or Ca10 treated with pronase (Ca10+pronase) for 18 h.

### Results

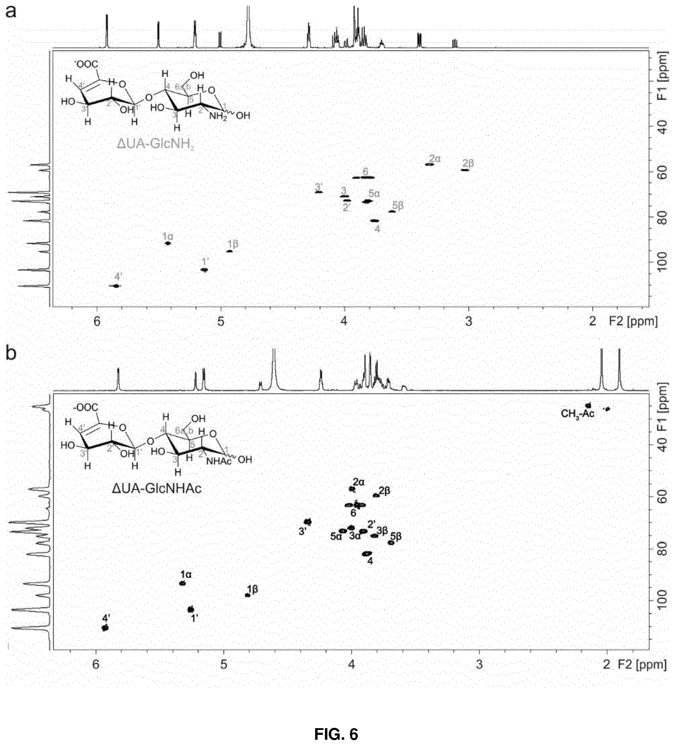

Ca10 derived from Ehrlich tumor cells is a heavily glycosylated high molecular weight structure, being the carbohydrate epitope recognized by mAb A10 sensitive to NaIO₄ oxidation **(****Figure 5a****),** which specifically cleaves bonds between vicinal carbons bearing unsubstituted hydroxyl or amino groups. Treatment of Ca10 with NalOa completely abolished the mAb A10 reactivity and resulted in significant degradation and thus molecular size reduction of the carbohydrate component as observed by NMR diffusion ordered spectroscopy (DOSY) **(****Figure 5a****).** To gain insight into the carbohydrate structure contained in Ca10 and considering that the NMR profiles of Ca10 saccharides are consistent with glycosaminoglycans (GAGs), different enzymes cleaving glycans at different positions were used **(Table 2).** Exoglycosidases had no effect on Ca10, nor had PNGase-F or O-Glycanase. Among all the tested enzymes, Ca10 was only degraded by the GAG-lyases of heparinase type, pointing to the glycan structure of Ca10 being a heparan sulfate-related GAG **(Table 2).** This was confirmed by the fact that treatment of Ca10 with heparinase II/III (HPSE treat.) completely abolished mAb A10 reactivity **(****Figure 5b****)** and also hydrolyzed the carbohydrate component into low molecular weight fragments as observed by diffusion NMR **(****Figure 5b****).** Likewise, the NMR profile of the major fraction of the resulting disaccharide fragments upon digestion matches the profiles of ΔUA-GIcNH2 and ΔUA-GIcHNHAc related to heparan sulfate-forming disaccharides **(****Figure 5c** **and** **Figures 6a, 6b****),** confirming that the main carbohydrate moiety of Ca10 is a GAG of heparan type.

**Table 2. Enzymatic digestion of Ca10 preparations.**

| | Activity | |
|---|---|---|
| Enzyme | Control | Ca10 |
| PNGase-F* | ++++ | - |
| O-Glycanase* | ++ | - |
| *β*-Acetylglucosaminidase 73A | ++++ | - |
| Glucosidases (*α*-, *β*-) | ++++ | - |
| Galactosidases (*α*-, *β*-) | ++++ | - |
| *α*-Manosidase | +++ | - |
| Sialidase | ++++ | - |
| *α*-Fucosidase | ++++ | - |
| Chitosanase 8B | ++++ | - |
| Heparinase III | +++ | ++ |
| Heparinase II | +++ | + |
| Chondroitinase ABC | ++++ | + |

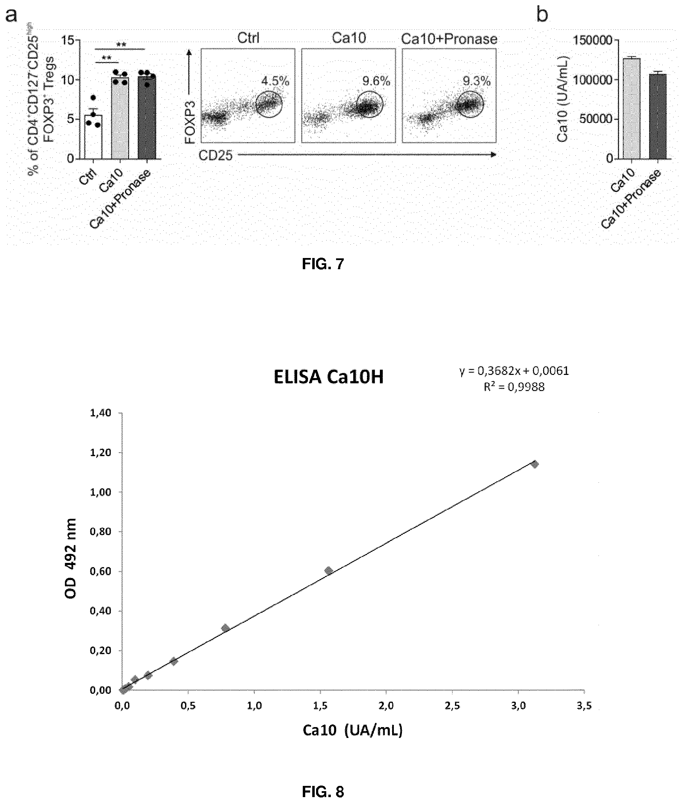

Supporting the above data, ET cells growth in the presence of specific inhibitors of heparan sulfate biosynthesis showed significantly lower Ca10 surface expression and lower levels of soluble Ca10 than untreated cells **(****Figure 5d****).** Oxidation of Ca10 with NalOa (Ca10 OX), heparinase treatment (HPSE treat.) or the presence of heparin abolished Ca10-mediated IL-10 production and significantly impaired PD-L1 expression in human DCs **(****Figures 5e, 5f****).** Similarly, the generation of CD4+CD127-CD25^{high}FOXP3⁺ Tregs by Ca10-stimulated DCs was significantly reduced when human DCs were stimulated with Ca10 previously subjected to oxidation with NaIO₄, treated with heparinase or in the presence of heparin **(****Figure 5g****).** Remarkably, human DCs stimulated with Ca10 previously treated with pronase (Ca10+pronase) induced similar percentage of Tregs than Ca10-stimulated DCs **(****Figure 7a****).** The treatment of Ca10 with pronase only slightly reduced mAb A10 reactivity without significant differences observed **(****Figure 7b****).** Collectively, these results demonstrate that the induction of Tregs is mediated by the presence of the epitope recognized by the mAb A10 located in the saccharide portion.

### Example 4. Patients with different carcinomas show higher serum levels of a human Ca10 homologue (Ca10H) than healthy controls.

### Material and Methods

Sera from patients diagnosed with prostate adenocarcinoma (n = 248), colorectal adenocarcinoma (n = 66) and other types of cancer (n = 71) at any stage of the disease, were obtained from the Central Laboratory of Hospital Clinico San Carlos (Madrid, Spain) during routine follow-up. Prostate cancer patients with (n=42) or without (n = 30) bone metastases from the Urology Department were selected to measure their serum levels of Ca10H, as described above for Ca10, and alkaline phosphatase by spectrophotometry. The serum samples from the healthy donors (n = 131) used as controls were from the Blood Donor Unit of the Hospital Clínico San Carlos.

Ca10H was measured in human sera by a A10-based sandwich ELISA. In this assay, purified mAb A10 (IgMκ) from culture supernatants of the mAb A10-producing hybridoma was used as capture and detection antibody (HRP-labeled). Plates were coated overnight at 4°C with 5 µg/mL of mAb A10 in 0.05 M carbonate-bicarbonate buffer. Then, they were washed with PBS 0.25% Tween-20 (PBS-Tw) and incubated 2 h at room temperature (RT) with the samples diluted in PBS-Tw. After a wash step, a dilution of HRP-mAb A10 (1:1000 dilution) in PBS-Tw, 5% FBS was added and incubated for 2 h at RT. After a final washing, the peroxidase substrate (OPD, 0.63 mg/mL) was added in 0.1 M citrate buffer with 0.03% H_0₂ - pH 5.5. The enzymatic reaction was allowed to develop for 30 min and stopped by adding a 10% HCl solution. Ca10H concentration was expressed as arbitrary units (AU) per mL by extrapolating the OD (495 nm) to a reference curve established with Ca10. The detection and quantitation limits were established in 0.03 and 0.05 AU/mL respectively, with a linear range between 0.03 to 1.56 AU/mL (R²>0,98). Sensivity and specifity (% of recovery=99.85) assays were performed to validate its use for Ca10 measurement **(****Figure 8****)**

### Results

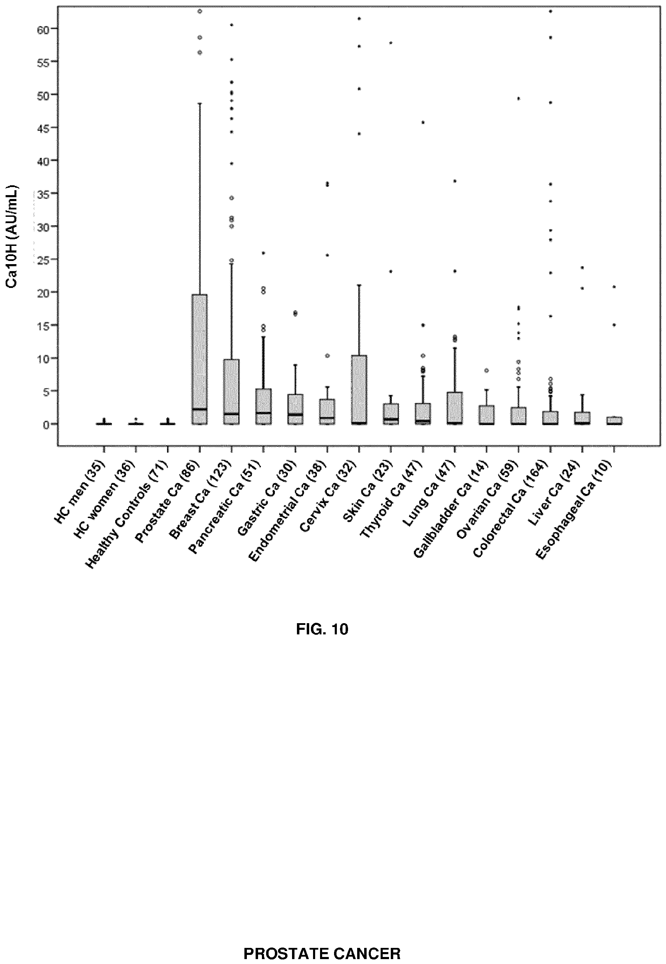

To assess the presence of circulating human Ca10 homologue (Ca10H) and to quantify its serum levels in cancer patients and healthy controls, the same ELISA as for murine Ca10 was used as described in material and methods **(****Figure 9a****).** Sera from patients with different types of cancer under follow-up at any stage of the disease were tested. As shown, the Ca10H levels in patients with prostate (n=248), colorectal (n=66) or other cancer types (n=71) were significantly increased compared to those detected in healthy donors (n=131) **(****Figure 9b****).** To gain preliminary insight into the potential clinical relevance of Ca10H levels in cancer patients, different serum samples from prostate cancer patients with (n=42) or without (n=30) bone metastases were tested. As shown in **Figure 9c****,** prostate cancer patients with bone metastases showed higher levels of Ca10H than those without metastases. Supporting these data, serum Ca10H levels correlated with serum alkaline phosphatase in patients with bone metastases, a well-recognized bone turnover biomarker **(****Figure 9d****).** Notably, treatment of different sera from prostate cancer patients with heparinase, but not with sialidase used as control, completely abolished Ca10H detection in those sera **(****Figure 9e****),** verifying the relationship of human Ca10H to murine Ca10 and to heparan sulfate, at least in serum from prostate cancer patients. **Figure 10** shows the serum Ca10H levels in a second cohort of cancer patients, distinct from those presented in **Figure 9a****.** This confirms that the mean values of Ca10H levels are elevated in almost all patients with epithelial cancers, compared to those in healthy controls **(****Figure 10****).**

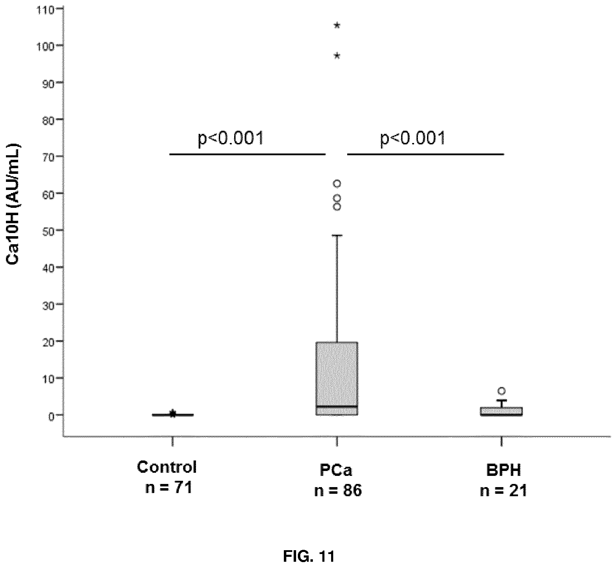

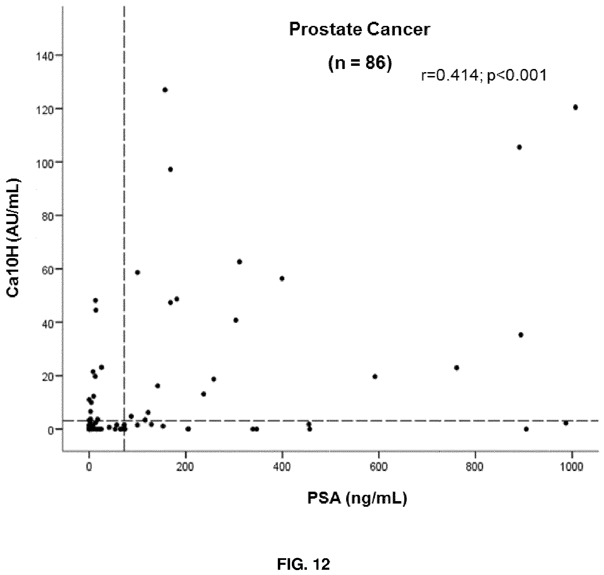

In the case of prostate cancer, serum Ca10H values were significantly higher compared to both healthy controls and patients with benign prostatic hyperplasia **(****Figure 11****).** The correlation between Ca10H and serum PSA (prostate-specific antigen) in this type of cancer was weak (r<0.5), yet still statistically significant **(****Figure 12****).**

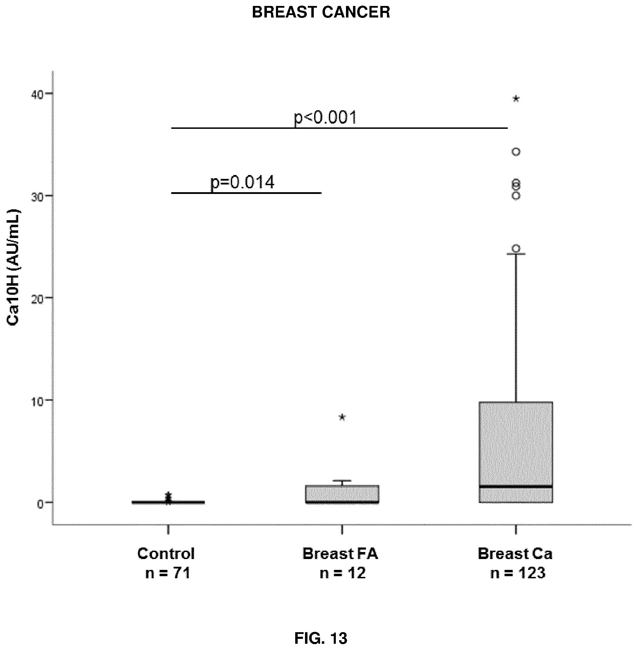

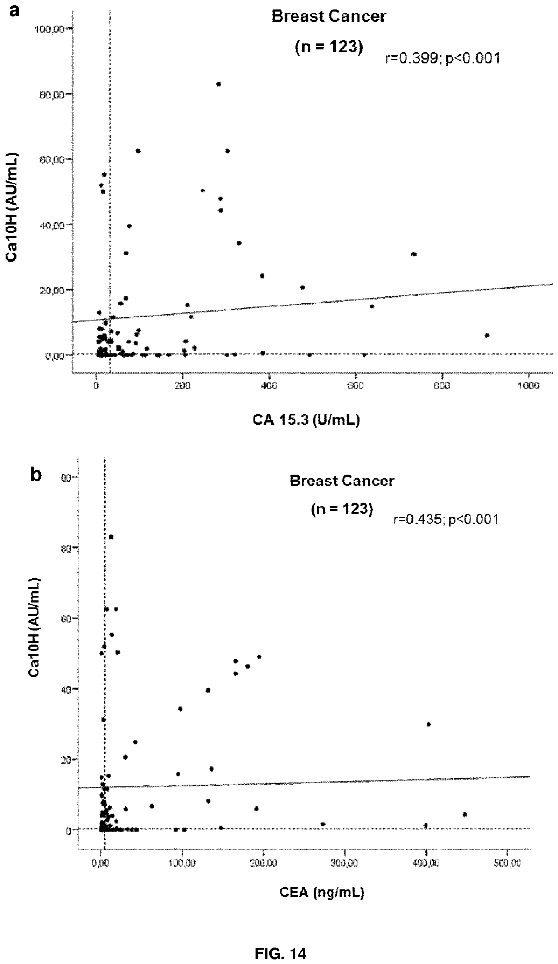

In breast cancer, mean serum Ca10H levels were also elevated relative to healthy controls **(****Figure 13****).** In the case of fibroadenomas, a benign breast tumor, these levels were also significantly higher than in healthy controls, although to a lesser extent **(****Figure 13****).** The correlation between the two commonly used tumor markers in breast cancer (CA 15.3 and CEA) and Ca10H was weak (r<0.5) in both cases, although statistically significant **(****Figure 14****).**

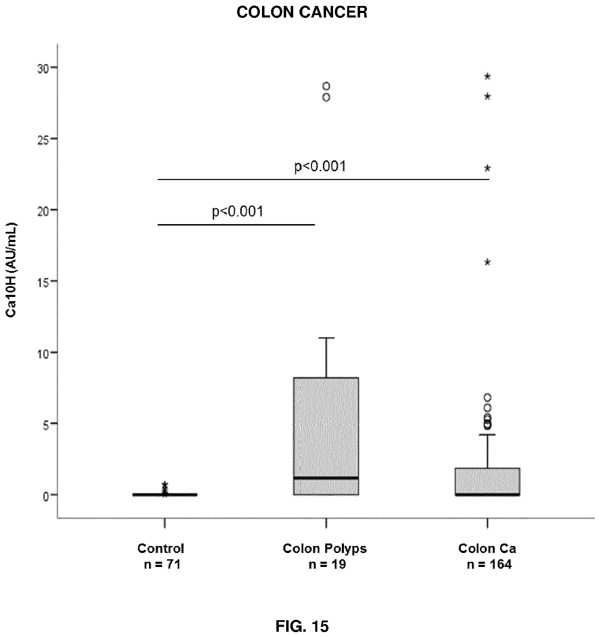

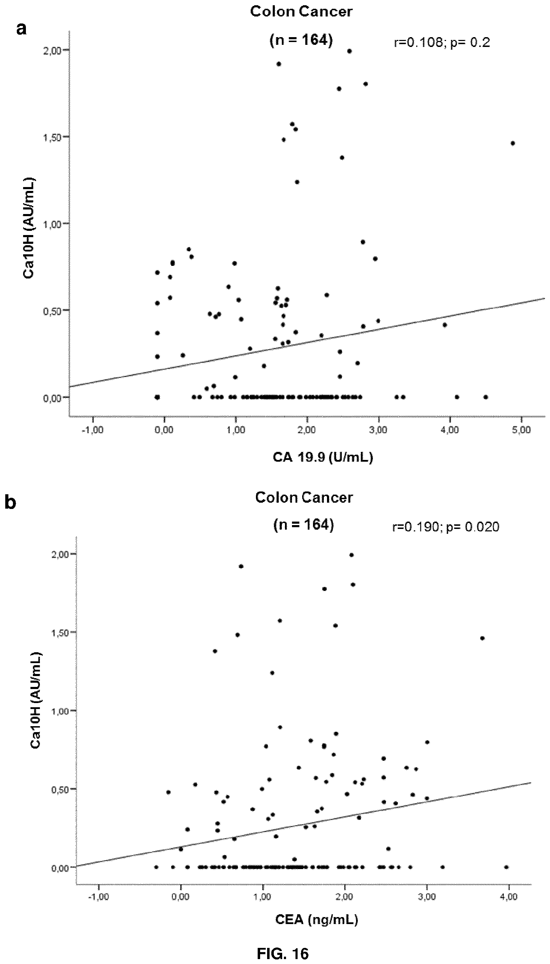

In patients with colorectal cancer, serum Ca10H levels were elevated compared to healthy controls **(****Figure 15****).** These levels were also increased in patients with colon polyposis compared to controls **(****Figure 15****).** No correlation was observed between the two commonly used tumor markers in colorectal cancer (CA 19.9 and CEA) and Ca10H **(****Figure 16****).**

## Claims

1. An *in vitro* method for detecting cancer in a subject, comprising:
(a) quantifying the tumoral carbohydrate Ca10 (Ca10) levels in a biological sample isolated from the subject; and
(b) comparing the Ca10 levels obtained in step (a) with a control level,
Wherein if the Ca10 levels in the subject are increased with respect to the control levels, then the subject suffers from cancer.

2. An *in vitro* method for predicting the clinical outcome of a subject suffering from cancer, or for monitoring the treatment of a subject suffering from cancer, comprising
(a) quantifying the tumoral carbohydrate Ca10 (Ca10) levels in a biological sample isolated from the subject; and
(b) comparing the Ca10 levels obtained in step (a) with a control level,
Wherein if the Ca10 levels in the subject are increased with respect to the control level, then
- the clinical outcome of the subject is negative and either the subject is going to suffer from metastasis or the tumoral mass is going to increase; or
- the treatment is not effective.

3. The method according to claim 1 or 2, wherein the cancer is a carcinoma or an adenocarcinoma.

4. The method according to any one of claims 1 to 3, wherein the cancer is prostate cancer, cervix cancer, breast cancer, pancreatic cancer, lung cancer, gastric cancer, endometrial cancer, skin cancer, thyroid cancer, bladder cancer, ovarian cancer, lever cancer, esophageal cancer, or colorectal cancer.

5. The method according to any one of claims 1 to 4, wherein the biological sample is blood, serum, plasma, pleural liquid, bronchioalveolar liquid, peritoneal liquid, faeces, urine, sputum or tissue biopsy specimens.

6. Use of a kit for detecting cancer *in vitro,* or for predicting *in vitro* the clinical outcome of a subject suffering from cancer, or for monitoring *in vitro* the treatment of a subject suffering from cancer, wherein the kit comprises elements for quantifying the Ca10 levels in a biological sample of a subject.

7. Use of a kit for detecting cancer *in vitro,* or for predicting *in vitro* the clinical outcome of a subject suffering from cancer, or for monitoring *in vitro* the treatment of a subject suffering from cancer, according to claim 6, wherein the cancer is a carcinoma or an adenocarcinoma.

8. Use of a kit for detecting cancer *in vitro,* or for predicting *in vitro* the clinical outcome of a subject suffering from cancer, or for monitoring *in vitro* the treatment of a subject suffering from cancer, according to claim 6 or 7, wherein the cancer is prostate cancer, cervix cancer, breast cancer, pancreatic cancer, lung cancer, gastric cancer, endometrial cancer, skin cancer, thyroid cancer, bladder cancer, ovarian cancer, lever cancer, esophageal cancer, or colorectal cancer.

9. Use of a kit for detecting cancer *in vitro,* or for predicting *in vitro* the clinical outcome of a subject suffering from cancer, or for monitoring *in vitro* the treatment of a subject suffering from cancer, according to any one of claims 6 to 8, wherein the biological sample is blood, serum or plasma pleural liquid, bronchioalveolar liquid, peritoneal liquid, faeces, urine, sputum or tissue biopsy specimens

10. Use of a kit for detecting cancer *in vitro,* or for predicting *in vitro* the clinical outcome of a subject suffering from cancer, or for monitoring *in vitro* the treatment of a subject suffering from cancer, according to any one of claims 6 to 9, wherein the elements are antibodies, or a fragment thereof, peptides or aptamers, all of them with affinity for Ca10.
